# EUROPEAN PATENT APPLICATION

(11) **EP 4 338 758 A1**
(43) Date of publication of application: **20.03.2024**
(21) Application number: 22196165.9
(22) Date of filing: 16.09.2022
(51) Int. Cl.: A61K 51/04

(54) **DIAGNOSTIC PET IMAGING OF CARDIAC AMYLOIDOSIS USING BISPHOSPHONATE TRACERS**

(71) Applicant: Beijing Lado Technology Co., Ltd., Beijing 100082 (CN)
(72) Inventor: HIGUCHI, Takahiro, 97080 Würzburg (DE); WERNER, Rudolf, 97080 Würzburg (DE)
(74) Representative: Maiwald GmbH

(57) **Abstract**

The present disclosure relates to a compound or pharmaceutically acceptable salt thereof comprising at least one bisphosphonate or bisphosphonate ester group, and a positron emitting radionuclide Po, and the use thereof in a method of diagnosis of amyloidosis, such as cardiac amyloidosis.

## Description

### Technical field

The present disclosure relates to compounds or pharmaceutically acceptable salts thereof comprising a bisphosphonate or bisphosphonate ester group and a positron emitting radionuclide, the preparation thereof, and the use thereof in a method of diagnosis, such as a method of diagnosis of cardiac amyloidosis.

### Background

Bisphosphonates are a class of drugs that prevent the loss of bone density, used to treat osteoporosis, high blood calcium levels due to cancer and similar diseases. Hence, they are the most commonly prescribed drugs used to treat osteoporosis.

The inventors of the present disclosure have surprisingly found that such bisphosphonate drugs can also be advantageously used in methods of diagnosis of cardiac amyloidosis.

As such, the drugs of the bisphosphonate class have in common, that they contain two phosphonate groups bound to only one carbon atom. In other words, all bisphosphonate drugs contain a structure of R'R"C-(PO(OX)₂)₂. When X is hydrogen, the bisphosphonate is present as a free acid in its neutral from; when X is a pharmaceutically acceptable cation, such as Na⁺, the bisphosphonate is present as a pharmaceutically acceptable salt in its charged from. When X is an alkyl or the like, the bisphosphonate is present as an ester, i.e. a prodrug. Upon hydrolysis of said ester in the body, the bisphosphonate is formed.

Without being bound by theory, a bisphosphonate group mimics the structure of pyrophosphate, and hence it can inhibit activation of enzymes that utilize pyrophosphate and the two phosphonate groups together coordinate calcium ions. The largest storage of calcium in the human body is in bones, so bisphosphonates accumulate to a high concentration only in bones.

Among the most prominent drugs in the bisphosphonate class are pamidronic acid, nedridronic acid, olpadronic acid, alendronic acid, ibandronic acid, risedronic acid, and zolendronic acid.

Due to the affinity of the bisphosphonate compounds to the bones, they can also be used in conjugation with a positron emitting radionucleotide as imaging probes for the bones.

US 2016/310621 A1 describes derivatives of risedronic acid and zolendronic acid conjugated with a positron emitting radionuclide as imaging probes for use in positron emission tomography of the bones and methods of synthesizing said bisphosphonate conjugates.

Cardiac amyloidosis is a condition of the heart with extracellular amyloid infiltration associated with abnormal cardiac function. Because of high morbidity and mortality of the cardiac amyloidosis, early and accurate detection of cardiac involvement and differentiation between the types are critical for appropriate therapy selection and patient's prognosis. The present invention provides novel bisphosphonate positron emission tomography (PET) tracers for accurate diagnosis of cardiac amyloidosis with highly sensitive and quantitative manner.

PET imaging has intrinsic advantages with high spatial-/time-resolution, sensitivity, and accuracy for detecting radionuclide concentration. Among available radionuclide for PET imaging, ¹⁸F-labeled tracers hold several advantages: 1) can be produced from a cyclotron and facilitated delivery from distant suppliers due to its longer half-life; 2) such delivery provides possibility of distribution to sites that are not allowed or short of facility to produce radiotracers on-site; 3) higher positron yield and lower positron energy that decreases image noise, improves contrast resolution, and maximizes the detection of subtle lesions; 4) the longer half-life allows for improved delayed imaging protocols.

### Summary of the invention

The present invention provides novel tracers and their use in method of diagnosis of cardiac amyloidosis by means of PET imaging technology and improves diagnostic accuracy compared to the scintigraphic techniques currently in use. From structure design point of view, the inventors provided different strategies to perform the radiolabeling of bisphosphonate compounds.

The inventors of the present disclosure have surprisingly found that radiolabeled tracers based on bisphosphonates or bisphosphonate esters can advantageously be used in a method of diagnosis of cardiac amyloidosis, i.e. a diseases that is not associated with the bones or other previously described applications of bisphosphonates.

In one aspect, the present disclosure relates to a compound or pharmaceutically acceptable salt thereof, wherein the compound or pharmaceutically acceptable salt thereof comprises at least one bisphosphonate or bisphosphonate ester group, and a positron emitting radionuclide Po, wherein Po is attached to the at least one bisphosphonate or bisphosphonate ester group.

In a further aspect, the present disclosure relates to a compound or pharmaceutically acceptable salt thereof for use in a method of diagnosis of cardiac amyloidosis, wherein the compound or pharmaceutically acceptable salt thereof comprises at least one bisphosphonate or bisphosphonate ester group, and a positron emitting radionuclide Po, wherein Po is attached to the at least one bisphosphonate or bisphosphonate ester group.

In a further aspect, the present disclosure relates to a method of preparation of a compound or pharmaceutically acceptable salt thereof.

### Description of the figures

- Fig. 1:: Time-activity curves of IVa
- Fig. 2:: Time-activity curves of ¹⁸F-NaF as control
- Fig. 3:: PET image 120 min after injection of IVa (left panel) and enlarged view of the heart area (right panel).
- Fig. 4:: PET image 120 min after injection of ¹⁸F-NaF as control
- Fig. 5:: Dynamic images PET images using IVa (top panel) and IVb (bottom panel) until 120 min after tracer injection.
- Fig. 6:: Comparison of uptake of IVa in human heart tissue with amyloidosis (top panel) and H&E staining on the same tissue (lower panel).

### Detailed Description

### The compound or pharmaceutically acceptable salt thereof

According to one embodiment, the present disclosure relates to a compound or pharmaceutically acceptable salt thereof, wherein the compound or pharmaceutically acceptable salt thereof comprises at least one bisphosphonate or bisphosphonate ester group, and a positron emitting radionuclide Po, wherein Po is attached to the at least one bisphosphonate or bisphosphonate ester group.

According to a preferred embodiment of the present disclosure, the compound or a pharmaceutically acceptable salt thereof, is a compound or pharmaceutically acceptable salt thereof of formula (I) 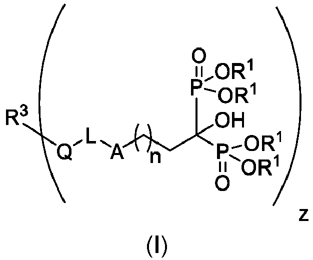 wherein z is an integer selected from the group consisting of 1, 2, and 3, preferably z is 1; and wherein each R¹ is independently hydrogen, a pharmaceutically acceptable cation, or a protection group; and wherein n is an integer selected from the group consisting of 0, 1, 2, and 3; and wherein A is -NR² , or (4 to 8 membered)heterocyclyl, or (4 to 8 membered)cyclyl, or squaramide, or (5 to 6 membered)heteroaryl, or phenyl; and wherein R² is hydrogen or linear or branched (C₁-C₆)alkyl; and wherein L and Q are absent; or wherein L is a divalent linker group; and wherein Q is -NH, -NR^{Q}, -N⁺R^{Q}₂X⁻, -O, *N,N*-substituted piperazine, or 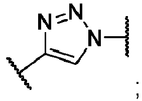 and wherein X⁻ is a pharmaceutically acceptable anion, preferably X⁻ is F⁻, Cl⁻, Br⁻, I⁻, OH⁻, tosylate, mesylate, triflate, trifluoroacetate, sulfate, acetate, tartrate, benzoate, oxylate, salicylate, or camphorsulfonate; more preferably Br; and wherein R^{Q} is each independently linear or branched (C₁-C₆)alkyl, preferably each R^{Q} is methyl; and wherein R³ is a group comprising the positron emitting radionuclide Po.

According to another preferred embodiment, the positron emitting radionuclide Po is ¹¹C, ¹³N, ¹⁵O, ¹⁸F, ⁶⁴Cu or ⁶⁸Ga. According to a further preferred embodiment, the positron emitting radionuclide Po is ¹¹C, ¹⁸F, or ⁶⁸Ga. In a further preferred embodiment, the positron emitting radionuclide is ¹⁸F or ⁶⁸Ga. In a further preferred embodiment, the positron emitting radionuclide is ¹⁸F. In a further preferred embodiment, the positron emitting radionuclide is ⁶⁸Ga. In a further preferred embodiment, the positron emitting radionuclide is ¹¹C.

According to another preferred embodiment, the present disclosure relates to a compound of formula (I) wherein z is 1. In other words, the compound has the structure (I.1)

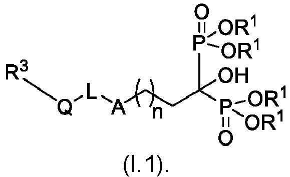

According to another preferred embodiment, the present disclosure relates to a compound of formula (I) wherein z is 2. In other words, the compound has the structure (I.2)

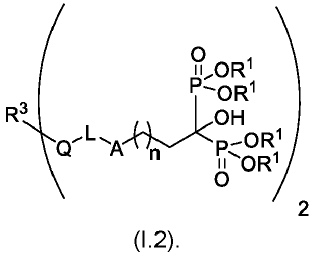

According to another preferred embodiment, the present disclosure relates to a compound of formula (I) wherein z is 3. In other words, the compound has the structure (I.3)

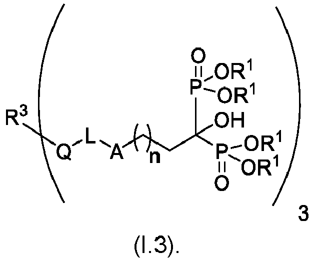

According to another preferred embodiment, the present disclosure relates to a compound of formula (I), (I.1), (I.2) or (I.3), wherein n is an integer selected from the group consisting of 0, 1, 2, and 3. According to another preferred embodiment, the present disclosure relates to a compound of formula (I), (I.1), (I.2) or (I.3), wherein n is 0. When n is 0, A is bound to the methylene group attached to the bisphosphonate or bisphosphonate ester group via a single bond. According to another preferred embodiment, the present disclosure relates to a compound of formula (I), (I.1), (I.2) or (I.3), wherein n is 1. According to another preferred embodiment, the present disclosure relates to a compound of formula (I), (I.1), (I.2) or (I.3), wherein n is 2. According to another preferred embodiment, the present disclosure relates to a compound of formula (I), (I.1), (I.2) or (I.3), wherein n is 3.

According to another preferred embodiment, the present disclosure relates to a compound of formula (I), (I.1), (I.2) or (I.3), wherein each R¹ is independently hydrogen, a pharmaceutically acceptable cation, or a protection group. In a further preferred embodiment, each R¹ is hydrogen. In another preferred embodiment, each R¹ is a pharmaceutically acceptable cation. In a further preferred embodiment, each R¹ is Na⁺, or Li⁺, or K⁺ or ½ Ca²⁺, or ½ Mg²⁺. In a further preferred embodiment, each R¹ is Na⁺. In a further preferred embodiment, each R¹ is Li⁺. In a further preferred embodiment, each R¹ is K⁺. In a further preferred embodiment, each R¹ is ½ Ca²⁺. In a further preferred embodiment, each R¹ is ½ Mg²⁺.

According to another preferred embodiment, the present disclosure relates to a compound of formula (I), (I.1), (I.2) or (I.3), wherein each R¹ is a protection group. According to another preferred embodiment, the present disclosure relates to a compound of formula (I), (I.1), (I.2) or (I.3), wherein each R¹ is a protection group selected from the group consisting of methyl, ethyl, n-propyl, i-propyl, n-butyl, i-butyl, t-butyl, -CH₂-O(CO)-CH₃, -CH₂-O(CO)-CH₂CH₃, -CH₂-O(CO)-CH(CH₃)₂, -CH₂-O(CO)-C(CH₃)₃, and 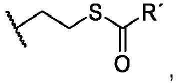 wherein R' is (C₁-C₁₀)alkyl, preferably methyl or ethyl. According to another preferred embodiment, the present disclosure relates to a compound of formula (I), (I.1), (I.2) or (I.3), wherein each R¹ is a protection group selected from the group consisting of methyl, ethyl, and i-propyl. According to another preferred embodiment, the present disclosure relates to a compound of formula (I), (I.1), (I.2) or (I.3), wherein each R¹ is methyl. According to another preferred embodiment, the present disclosure relates to a compound of formula (I), (I.1), (I.2) or (I.3), wherein each R¹ is i-propyl.

According to another preferred embodiment, the present disclosure relates to a compound of formula (I), (I.1), (I.2) or (I.3), wherein each R¹ is a protection group, wherein two R¹ attached to one phosphonate group together form a structure of 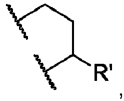 or 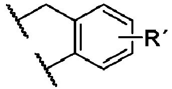 or 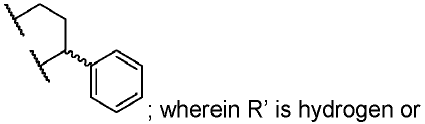 (C₁-C₁₀)alkyl.

According to another preferred embodiment, the present disclosure relates to a compound of formula (I), (I.1), (I.2) or (I.3), wherein A is -NR², or (4 to 8 membered)heterocyclyl, or (4 to 8 membered)cyclyl, or squaramide, or (5 to 6 membered)heteroaryl, or phenyl. According to another preferred embodiment, the present disclosure relates to a compound of formula (I), (I.1), (I.2) or (I.3), wherein A is (4 to 8 membered) heterocyclyl. According to another preferred embodiment, the present disclosure relates to a compound of formula (I), (I.1), (I.2) or (I.3), wherein A is a (4 to 8 membered)cyclyl. According to another preferred embodiment, the present disclosure relates to a compound of formula (I), (I.1), (I.2) or (I.3), wherein A is squaramide. According to another preferred embodiment, the present disclosure relates to a compound of formula (I), (I.1), (I.2) or (I.3), wherein A is (5 to 6 membered) heteroaryl. According to another preferred embodiment, the present disclosure relates to a compound of formula (I), (I.1), (I.2) or (I.3), wherein A is phenyl.

According to another preferred embodiment, the present disclosure relates to a compound of formula (I), (I.1), (I.2) or (I.3), wherein A is -NR², and wherein R² is hydrogen or linear or branched (C₁-C₆)alkyl. According to another preferred embodiment, the present disclosure relates to a compound of formula (I), (I.1), (I.2) or (I.3), wherein A is -NR², and wherein R² is hydrogen. According to another preferred embodiment, the present disclosure relates to a compound of formula (I), (I.1), (I.2) or (I.3), wherein A is -NR², and wherein R² is linear or branched (C₁-C₆)alkyl. According to another preferred embodiment, the present disclosure relates to a compound of formula (I), (I.1), (I.2) or (I.3), wherein A is -NR², and wherein R² is methyl. According to another preferred embodiment, the present disclosure relates to a compound of formula (I), (I.1), (I.2) or (I.3), wherein A is -NR², and wherein R² is ethyl.

It has been found by the inventors of the present disclosure that it is advantageous that group A contains a basic center, such as a secondary or tertiary amine group.

According to another preferred embodiment, the present disclosure relates to a compound of formula (I), (I.1), (I.2) or (I.3), wherein L and Q are absent. In other words, groups A and R³ are directly connected via a covalent bond.

According to another preferred embodiment, the present disclosure relates to a compound of formula (I), (I.1), (I.2) or (I.3), wherein L is a divalent linker group, and wherein Q is -NH, -NR^{Q}, -N⁺R^{Q}₂X⁻, -O, *N,N*-substituted piperazine, or 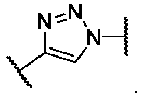 According to another preferred embodiment, the present disclosure relates to a compound of formula (I), (I.1), (I.2) or (I.3), wherein L is a divalent linker group, and wherein Q is -NH. According to another preferred embodiment, the present disclosure relates to a compound of formula (I), (I.1), (I.2) or (I.3), wherein L is a divalent linker group, and wherein Q is -O (oxygen). According to another preferred embodiment, the present disclosure relates to a compound of formula (I), (I.1), (I.2) or (I.3), wherein L is a divalent linker group, and wherein Q is *N,N-*substituted piperazine. According to another preferred embodiment, the present disclosure relates to a compound of formula (I), (I.1), (I.2) or (I.3), wherein L is a divalent linker group, and wherein Q is 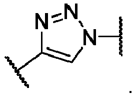 According to another preferred embodiment, the present disclosure relates to a compound of formula (I), (I.1), (I.2) or (I.3), wherein L is a divalent linker group, and wherein Q is -NR^{Q}, wherein R^{Q} is linear or branched (C₁-C₆)alkyl. According to another preferred embodiment, the present disclosure relates to a compound of formula (I), (I.1), (I.2) or (I.3), wherein L is a divalent linker group, and wherein Q is -NR^{Q}, wherein R^{Q} is methyl. According to another preferred embodiment, the present disclosure relates to a compound of formula (I), (I.1), (I.2) or (I.3), wherein L is a divalent linker group, and wherein Q is -N⁺R^{Q}₂X⁻, wherein R^{Q} is linear or branched (C₁-C₆)alkyl, and wherein X⁻ is a pharmaceutically acceptable anion. According to another preferred embodiment, the present disclosure relates to a compound of formula (I), (I.1), (I.2) or (I.3), wherein L is a divalent linker group, and wherein Q is -N⁺R^{Q}₂X⁻, wherein R^{Q} is methyl, and wherein X⁻ is F⁻, Cl⁻, Br, I⁻, OH⁻, tosylate, mesylate, triflate, trifluoroacetate, sulfate, acetate, tartrate, benzoate, oxylate, salicylate, or camphorsulfonate. According to another preferred embodiment, the present disclosure relates to a compound of formula (I), (I.1), (I.2) or (I.3), wherein L is a divalent linker group, and wherein Q is -N⁺R^{Q}₂X⁻, wherein R^{Q} is methyl, and wherein X- is Br⁻.

According to another preferred embodiment, the present disclosure relates to a compound of formula (I), (I.1), (I.2) or (I.3), wherein the moiety R³-Q-L-A- is 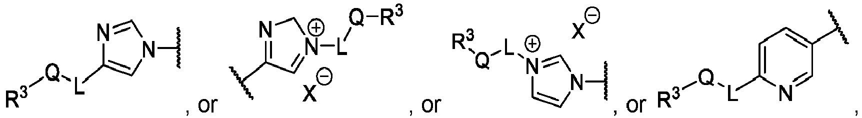 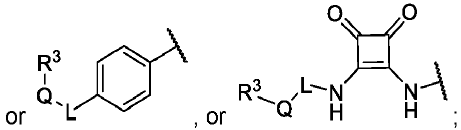 wherein X⁻ is a pharmaceutically acceptable anion. According to another preferred embodiment, the present disclosure relates to a compound of formula (I), (I.1), (I.2) or (I.3), wherein the moiety R³-Q-L-A- is

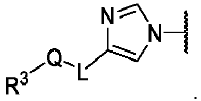

According to another preferred embodiment, the present disclosure relates to a compound of formula (I), (I.1), (I.2) or (I.3), wherein the moiety R³-Q-L-A- is 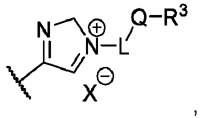 wherein X⁻ is F⁻, Cl⁻, Br⁻, I⁻, OH⁻, tosylate, mesylate, triflate, trifluoroacetate, sulfate, acetate, tartrate, benzoate, oxylate, salicylate, or camphorsulfonate.

According to another preferred embodiment, the present disclosure relates to a compound of formula (I), (I.1), (I.2) or (I.3), wherein the moiety R³-Q-L-A- is 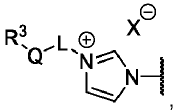 wherein X⁻ is F⁻, Cl⁻, Br⁻, I⁻, OH⁻, tosylate, mesylate, triflate, trifluoroacetate, sulfate, acetate, tartrate, benzoate, oxylate, salicylate, or camphorsulfonate.

According to another preferred embodiment, the present disclosure relates to a compound of formula (I), (I.1), (I.2) or (I.3), wherein the moiety R³-Q-L-A- is

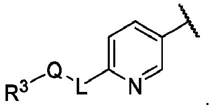

According to another preferred embodiment, the present disclosure relates to a compound of formula (I), (I.1), (I.2) or (I.3), wherein the moiety R³-Q-L-A- is

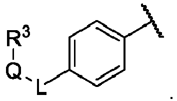

According to another preferred embodiment, the present disclosure relates to a compound of formula (I), (I.1), (I.2) or (I.3), wherein the moiety R³-Q-L-A- is

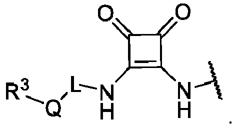

According to another preferred embodiment, the present disclosure relates to a compound of formula (I), (I.1), (I.2) or (I.3), wherein the moiety R³-Q-L- is 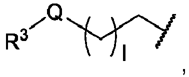 wherein
I is an integer selected from the group consisting of 1, 2, 3, 4, 5, 6, and 7, preferably wherein I is 1, 2, or 3; and wherein Q is-NH, -NR^{Q}, -N⁺R^{Q}₂X⁻, -O, *N,N-*substituted piperazine, or 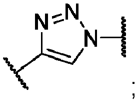 wherein X⁻ is a pharmaceutically acceptable anion, preferably X⁻ is F⁻, Cl⁻, Br⁻, I⁻, OH⁻, tosylate, mesylate, triflate, trifluoroacetate, sulfate, acetate, tartrate, benzoate, oxylate, salicylate, or camphorsulfonate; more preferably Br⁻; and wherein R^{Q} is each independently linear or branched (C₁-C₆)alkyl, preferably each R^{Q} is methyl.

According to another preferred embodiment, the present disclosure relates to a compound of formula (I), (I.1), (I.2) or (I.3), wherein the moiety R³-Q-L- is -CH₂-CH₂-NH-R³. According to another preferred embodiment, the present disclosure relates to a compound of formula (I), (I.1), (I.2) or (I.3), wherein the moiety R³-Q-L- is -CH₂-CH₂-CH₂-NH-R³. According to another preferred embodiment, the present disclosure relates to a compound of formula (I), (I.1), (I.2) or (I.3), wherein the moiety R³-Q-L- is -CH₂-CH₂-CH₂-CH₂-NH-R³.

According to another preferred embodiment, the present disclosure relates to a compound of formula (I), (I.1), (I.2) or (I.3), wherein the moiety R³-Q-L- is -CH₂-CH₂-N(CH₃)-R³. According to another preferred embodiment, the present disclosure relates to a compound of formula (I), (I.1), (I.2) or (I.3), wherein the moiety R³-Q-L- is -CH₂-CH₂-CH₂-N(CH₃)-R³. According to another preferred embodiment, the present disclosure relates to a compound of formula (I), (I.1), (I.2) or (I.3), wherein the moiety R³-Q-L- is -CH₂-CH₂-CH₂-CH₂-N(CH₃)-R³.

According to another preferred embodiment, the present disclosure relates to a compound of formula (I), (I.1), (I.2) or (I.3), wherein the moiety R³-Q-L- is -CH₂-CH₂-N⁺(CH₃)₂-R³ X⁻, wherein X⁻ is a pharmaceutically acceptable anion, preferably X⁻ is F⁻, Cl⁻, Br⁻, I⁻, OH⁻, tosylate, mesylate, triflate, trifluoroacetate, sulfate, acetate, tartrate, benzoate, oxylate, salicylate, or camphorsulfonate; more preferably Br⁻. According to another preferred embodiment, the present disclosure relates to a compound of formula (I), (I.1), (I.2) or (I.3), wherein the moiety R³-Q-L- is -CH₂-CH₂-CH₂-N⁺(CH₃)₂-R³ X⁻, wherein X⁻ is a pharmaceutically acceptable anion, preferably X⁻ is F⁻, Cl⁻, Br, I⁻, OH⁻, tosylate, mesylate, triflate, trifluoroacetate, sulfate, acetate, tartrate, benzoate, oxylate, salicylate, or camphorsulfonate; more preferably Br⁻. According to another preferred embodiment, the present disclosure relates to a compound of formula (I), (I.1), (I.2) or (I.3), wherein the moiety R³-Q-L- is -CH₂-CH₂-CH₂-CH₂-N⁺(CH₃)₂-R³ X⁻, wherein X⁻ is a pharmaceutically acceptable anion, preferably X⁻ is F⁻, Cl⁻, Br⁻, I⁻, OH⁻, tosylate, mesylate, triflate, trifluoroacetate, sulfate, acetate, tartrate, benzoate, oxylate, salicylate, or camphorsulfonate; more preferably Br⁻.

According to another preferred embodiment, the present disclosure relates to a compound of formula (I), (I.1), (I.2) or (I.3), wherein L is a linear or branched (C₁-C₁₀) alkyl. According to another preferred embodiment, the present disclosure relates to a compound of formula (I), (I.1), (I.2) or (I.3), wherein L is a substituted or unsubstituted aryl. According to another preferred embodiment, the present disclosure relates to a compound of formula (I), (I.1), (I.2) or (I.3), wherein L is a substituted or unsubstituted heteroaryl. According to another preferred embodiment, the present disclosure relates to a compound of formula (I), (I.1), (I.2) or (I.3), wherein L is a (5 to 8 membered)heterocyclyl According to another preferred embodiment, the present disclosure relates to a compound of formula (I), (I.1), (I.2) or (I.3), wherein L is a or (5 to 8 membered)heterocyclyl substituted with (C₁-C₁₀)alkyl. According to another preferred embodiment, the present disclosure relates to a compound of formula (I), (I.1), (I.2) or (I.3), wherein L is a dipeptide. According to another preferred embodiment, the present disclosure relates to a compound of formula (I), (I.1), (I.2) or (I.3), wherein L is a tripeptide. According to another preferred embodiment, the present disclosure relates to a compound of formula (I), (I.1), (I.2) or (I.3), wherein L is a PEG-linker.

According to another preferred embodiment, the present disclosure relates to a compound of formula (I), (I.1), (I.2) or (I.3), wherein Q is-NH. According to another preferred embodiment, the present disclosure relates to a compound of formula (I), (I.1), (I.2) or (I.3), wherein Q is O. According to another preferred embodiment, the present disclosure relates to a compound of formula (I), (I.1), (I.2) or (I.3), wherein Q is *N,N*-substituted piperazine. According to another preferred embodiment, the present disclosure relates to a compound of formula (I), (I.1), (I.2) or (I.3), wherein Q is -NR^{Q}, wherein R^{Q} is linear or branched (C₁-C₆)alkyl, preferably R^{Q} is methyl. According to another preferred embodiment, the present disclosure relates to a compound of formula (I), (I.1), (I.2) or (I.3), wherein Q is -N⁺R^{Q}₂X⁻, wherein X⁻ is a pharmaceutically acceptable anion, preferably X⁻ is F⁻, Cl⁻, Br⁻, I⁻, OH⁻, tosylate, mesylate, triflate, trifluoroacetate, sulfate, acetate, tartrate, benzoate, oxylate, salicylate, or camphorsulfonate; more preferably Br⁻; and wherein R^{Q} is each independently linear or branched (C₁-C₆)alkyl, preferably each R^{Q} is methyl. According to another preferred embodiment, the present disclosure relates to a compound of formula (I), (I.1), (I.2) or (I.3), wherein Q is

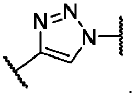

According to another preferred embodiment, the present disclosure relates to a compound of formula (I), (I.1), (I.2) or (I.3), wherein the moiety R³-Q-L- is

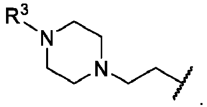

According to another preferred embodiment, the present disclosure relates to a compound of formula (I), (I.1), (I.2) or (I.3), wherein the moiety R³-Q-L- is

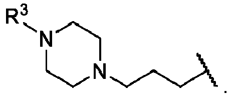

According to another preferred embodiment, the present disclosure relates to a compound of formula (I), (I.1), (I.2) or (I.3), wherein the moiety R³-Q-L- is

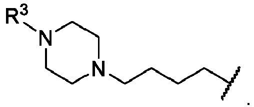

According to a further embodiment, the present disclosure relates to a compound of formula (I), (I.1), (I.2) or (I.3), wherein R³ is a group comprising the positron emitting radionuclide Po. According to a further embodiment, the present disclosure relates to a compound of formula (I), (I.1), (I.2) or (I.3), wherein R³ is 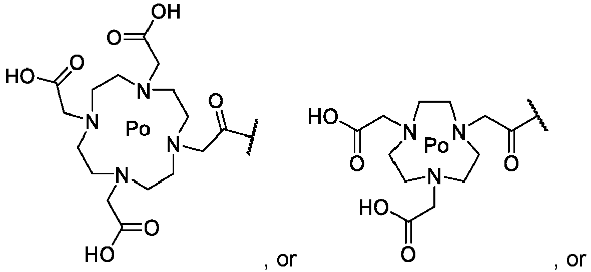 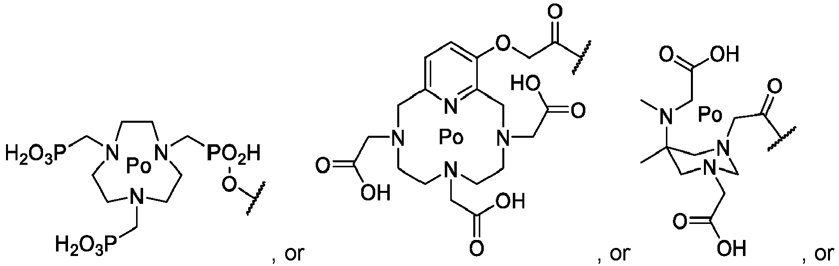 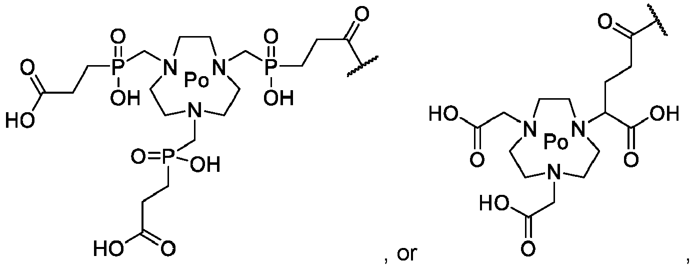 or 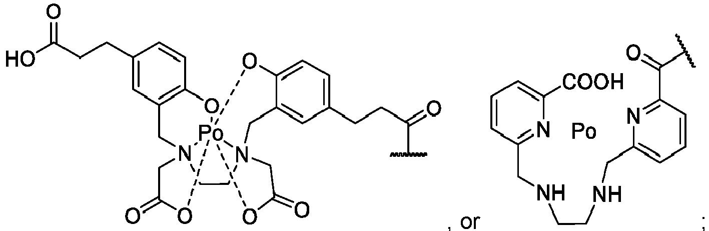 and wherein Po is ⁶⁴Cu or ⁶⁸Ga.

According to a further embodiment, the present disclosure relates to a compound of formula (I), (I.1), (I.2) or (I.3), wherein R³ is 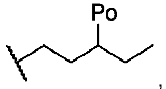 wherein Po is ¹⁸F or -O¹¹CH₃, preferably ¹⁸F.

According to a further embodiment, the present disclosure relates to a compound of formula (I), (I.1), (I.2) or (I.3), wherein R³ is -¹¹CH₃.

According to a further embodiment, the present disclosure relates to a compound of formula (I), (I.1), (I.2) or (I.3), wherein R³ is 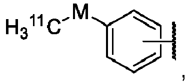 wherein M is O, or-NH, or CH₂O.

According to a further embodiment, the present disclosure relates to a compound of formula (I), (I.1), (I.2) or (I.3), wherein R³ is

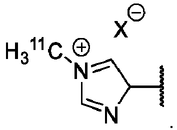

According to another preferred embodiment, the present disclosure relates to a compound of formula (I), (I.1), (I.2) or (I.3), wherein R³ is

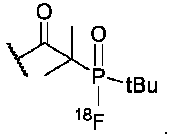

According to a further embodiment, the present disclosure relates to a compound of formula (I), (I.1), (I.2) or (I.3), wherein R³ is

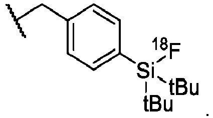

According to a further embodiment, the present disclosure relates to a compound of formula (I), (I.1), (I.2) or (I.3), wherein R³ is

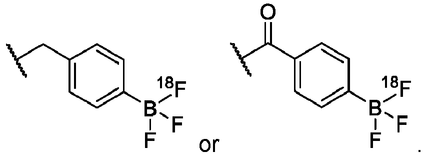

According to a further embodiment, the present disclosure relates to a compound of formula (I), (I.1), (I.2) or (I.3), wherein R³ is

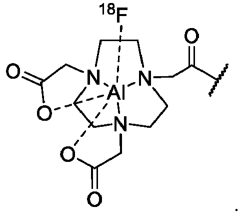

According to a further embodiment, the present disclosure relates to a compound of formula (I), (I.1), (I.2) or (I.3), wherein R³ is

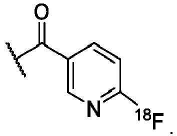

According to a further embodiment, the present disclosure relates to a compound of formula (I), (I.1), (I.2) or (I.3), wherein R³ is

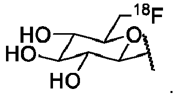

### Derivatives of ibandronic acid

According to one embodiment the present invention relates to a derivative of ibandronic acid. In other words, according to one embodiment the present invention relates to a compound of formula (I.a), wherein n is 1, A is -NR², and L and Q are absent. Accordingly, the present disclosure relates to a compound or pharmaceutically acceptable salt thereof of formula (II) 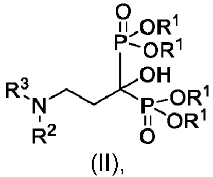 wherein R³ is a group comprising the positron emitting radionuclide Po; and wherein R² is hydrogen or linear or branched (C₁-C₆)alkyl; and wherein each R¹ is independently selected from the group consisting of hydrogen, linear or branched (C₁-C₆)alkyl, and -CH₂-O-CO-O-(C₁-C₆)alkyl.

According to a preferred embodiment, the present disclosure relates to a compound or pharmaceutically acceptable salt thereof of formula (II), wherein R³ is 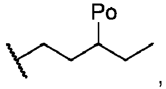 wherein Po is ¹⁸F or -O¹¹CH₃, preferably ¹⁸F; or

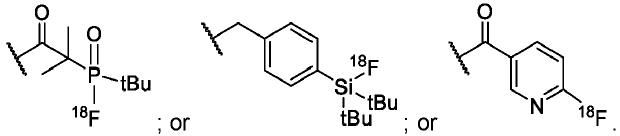

According to a preferred embodiment, the present disclosure relates to a compound or pharmaceutically acceptable salt thereof of formula (II), wherein R³ is 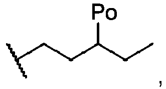 wherein Po is ¹⁸F or -O¹¹CH₃. According to a preferred embodiment, the present disclosure relates to a compound or pharmaceutically acceptable salt thereof of formula (II), wherein R³ is

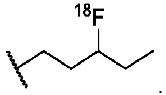

According to a preferred embodiment, the present disclosure relates to a compound or pharmaceutically acceptable salt thereof of formula (II), wherein R² is hydrogen. According to a preferred embodiment, the present disclosure relates to a compound or pharmaceutically acceptable salt thereof of formula (II), wherein R² is methyl.

According to a preferred embodiment, the present disclosure relates to a compound or pharmaceutically acceptable salt thereof of formula (II), wherein each R¹ is hydrogen, methyl, ethyl, i-propyl, or -CH₂-O-CO-O-C(CH₃)₃. According to a preferred embodiment, the present disclosure relates to a compound or pharmaceutically acceptable salt thereof of formula (II), wherein each R¹ is hydrogen. According to a preferred embodiment, the present disclosure relates to a compound or pharmaceutically acceptable salt thereof of formula (II), wherein each R¹ is methyl. According to a preferred embodiment, the present disclosure relates to a compound or pharmaceutically acceptable salt thereof of formula (II), wherein each R¹ is ethyl. According to a preferred embodiment, the present disclosure relates to a compound or pharmaceutically acceptable salt thereof of formula (II), wherein each R¹ is i-propyl. According to a preferred embodiment, the present disclosure relates to a compound or pharmaceutically acceptable salt thereof of formula (II), wherein each R¹ is -CH₂-O-CO-O-C(CH₃)₃.

According to a preferred embodiment, the present disclosure relates to a compound or pharmaceutically acceptable salt thereof of formula (IIa)

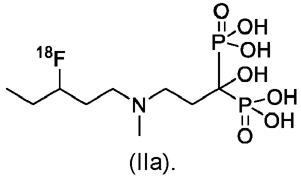

According to a preferred embodiment, the present disclosure relates to a compound or pharmaceutically acceptable salt thereof of formula (IIb) 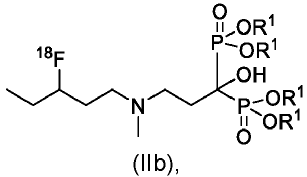 wherein each R¹ is methyl, ethyl, i-propyl, or -CH₂-O-CO-O-C(CH₃)₃.

According to a preferred embodiment, the present disclosure relates to a compound or pharmaceutically acceptable salt thereof of formula (IIc) 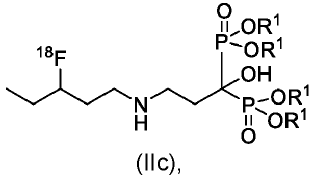 wherein each R¹ is methyl, ethyl, i-propyl, or -CH₂-O-CO-O-C(CH₃)₃.

According to another preferred embodiment, the present disclosure relates to a compound or pharmaceutically acceptable salt thereof of formula (IId)

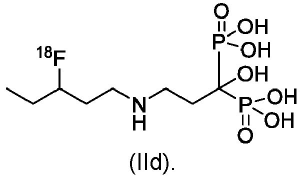

According to another preferred embodiment, the present disclosure relates to a compound or pharmaceutically acceptable salt thereof of formula (IIe)

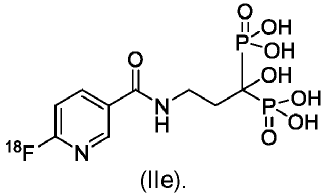

According to another preferred embodiment, the present disclosure relates to a compound or pharmaceutically acceptable salt thereof of formula (IIf)

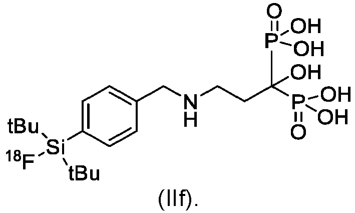

According to another preferred embodiment the present invention relates to a compound of formula (I.a), wherein n is 1, and A is -NR².

According to a preferred embodiment, the present disclosure relates to a compound or pharmaceutically acceptable salt thereof of formula (III) 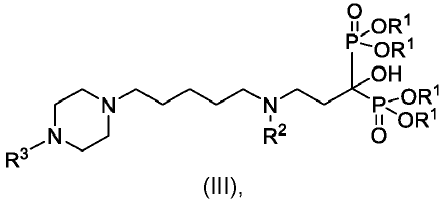 wherein R³ is a group comprising the positron emitting radionuclide Po and wherein R² is hydrogen or linear or branched (C₁-C₆)alkyl; and wherein each R¹ is independently selected from the group consisting of hydrogen, linear or branched (C₁-C₆)alkyl, and -CH₂-O-CO-O-(C₁-C₆)alkyl.

According to a preferred embodiment, the present disclosure relates to a compound or pharmaceutically acceptable salt thereof of formula (III), wherein R³ is 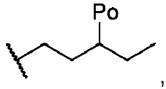 wherein Po is ¹⁸F or -O¹¹CH₃, preferably ¹⁸F; or

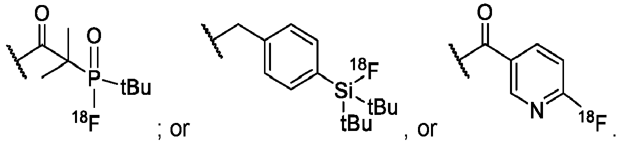

According to a preferred embodiment, the present disclosure relates to a compound or pharmaceutically acceptable salt thereof of formula (III), wherein R² is hydrogen. According to a preferred embodiment, the present disclosure relates to a compound or pharmaceutically acceptable salt thereof of formula (III), wherein R² is methyl.

According to a preferred embodiment, the present disclosure relates to a compound or pharmaceutically acceptable salt thereof of formula (III), wherein each R¹ is hydrogen, methyl, ethyl, i-propyl, or -CH₂-O-CO-O-C(CH₃)₃. According to a preferred embodiment, the present disclosure relates to a compound or pharmaceutically acceptable salt thereof of formula (III), wherein each R¹ is hydrogen. According to a preferred embodiment, the present disclosure relates to a compound or pharmaceutically acceptable salt thereof of formula (III), wherein each R¹ is methyl. According to a preferred embodiment, the present disclosure relates to a compound or pharmaceutically acceptable salt thereof of formula (III), wherein each R¹ is ethyl. According to a preferred embodiment, the present disclosure relates to a compound or pharmaceutically acceptable salt thereof of formula (III), wherein each R¹ is i-propyl. According to a preferred embodiment, the present disclosure relates to a compound or pharmaceutically acceptable salt thereof of formula (III), wherein each R¹ is -CH₂-O-CO-O-C(CH₃)₃.

According to a preferred embodiment, the present disclosure relates to a compound or pharmaceutically acceptable salt thereof of formula (III), wherein R³ is 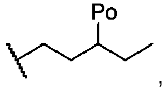 wherein Po is ¹⁸F or -O¹¹CH₃. According to a preferred embodiment, the present disclosure relates to a compound or pharmaceutically acceptable salt thereof of formula (III), wherein R³ is 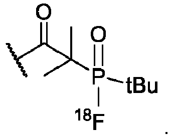 According to a preferred embodiment, the present disclosure relates to a compound or pharmaceutically acceptable salt thereof of formula (III), wherein R³ is

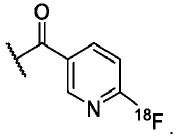

According to a further preferred embodiment, the present disclosure relates to a compound or pharmaceutically acceptable salt thereof of formula (IIIa)

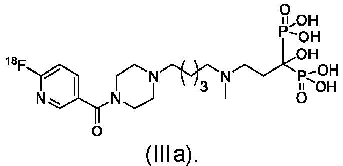

According to a further preferred embodiment, the present disclosure relates to a compound or pharmaceutically acceptable salt thereof of formula (IIIb)

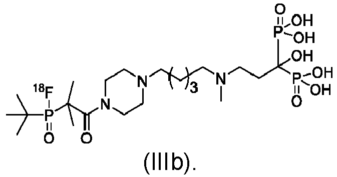

According to another preferred embodiment, the present disclosure relates to a compound or pharmaceutically acceptable salt thereof of formula (IIIc) 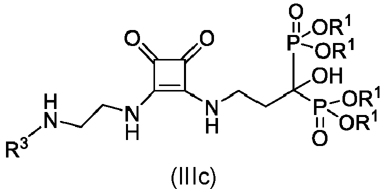 wherein R³ is a group comprising the positron emitting radionuclide Po; and wherein each R¹ is independently selected from the group consisting of hydrogen, linear or branched (C₁-C₆)alkyl, and -CH₂-O-CO-O-(C₁-C₆)alkyl.

According to another preferred embodiment, the present disclosure relates to a compound or pharmaceutically acceptable salt thereof of formula (IIIc) wherein R³ is 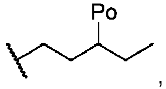 wherein Po is ¹⁸F or -O¹¹CH₃, preferably ¹⁸F; or 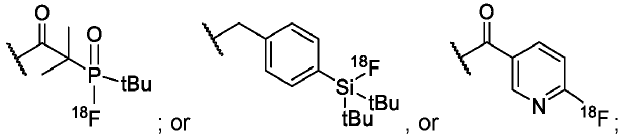 and wherein each R¹ is independently selected from the group consisting of hydrogen, linear or branched (C₁-C₆)alkyl, and -CH₂-O-CO-O-(C₁-C₆)alkyl.

### Derivatives of alendronic acid

According to one embodiment the present invention relates to a derivative of alendronic acid. In other words, according to one embodiment the present invention relates to a compound of formula (I.a), wherein n is 2, A is -NR², and L and Q are absent. According to a preferred embodiment, the present disclosure relates to a compound or pharmaceutically acceptable salt thereof of formula (IV) 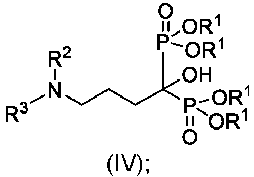 wherein R³ is a group comprising the positron emitting radionuclide Po; and wherein R² is hydrogen or linear or branched (C₁-C₆)alkyl; and wherein each R¹ is independently selected from the group consisting of hydrogen, linear or branched (C₁-C₆)alkyl, and -CH₂-O-CO-O-(C₁-C₆)alkyl.

According to a preferred embodiment, the present disclosure relates to a compound or pharmaceutically acceptable salt thereof of formula (IV), wherein R³ is 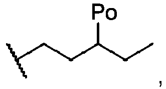 wherein Po is ¹⁸F or -O¹¹CH₃, preferably ¹⁸F; or 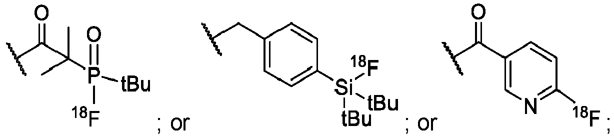 and wherein R² is hydrogen or linear or branched (C₁-C₆)alkyl; and wherein each R¹ is independently selected from the group consisting of hydrogen, linear or branched (C₁-C₆)alkyl, and -CH₂-O-CO-O-(C₁-C₆)alkyl.

According to a preferred embodiment, the present disclosure relates to a compound or pharmaceutically acceptable salt thereof of formula (IV), wherein R³ is 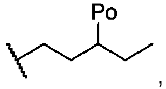 wherein Po is ¹⁸F or -O¹¹CH₃. According to a preferred embodiment, the present disclosure relates to a compound or pharmaceutically acceptable salt thereof of formula (IV), wherein R³ is 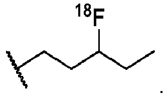 According to a preferred embodiment, the present disclosure relates to a compound or pharmaceutically acceptable salt thereof of formula (IV), wherein R³ is 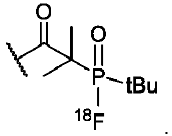 According to a preferred embodiment, the present disclosure relates to a compound or pharmaceutically acceptable salt thereof of formula (IV), wherein R³ is

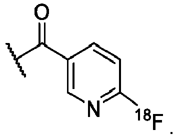

According to a preferred embodiment, the present disclosure relates to a compound or pharmaceutically acceptable salt thereof of formula (IV), wherein R² is hydrogen. According to a preferred embodiment, the present disclosure relates to a compound or pharmaceutically acceptable salt thereof of formula (IV), wherein R² is methyl.

According to a preferred embodiment, the present disclosure relates to a compound or pharmaceutically acceptable salt thereof of formula (IV), wherein each R¹ is hydrogen, methyl, ethyl, i-propyl, or -CH₂-O-CO-O-C(CH₃)₃. According to a preferred embodiment, the present disclosure relates to a compound or pharmaceutically acceptable salt thereof of formula (IV), wherein each R¹ is hydrogen. According to a preferred embodiment, the present disclosure relates to a compound or pharmaceutically acceptable salt thereof of formula (IV), wherein each R¹ is methyl. According to a preferred embodiment, the present disclosure relates to a compound or pharmaceutically acceptable salt thereof of formula (IV), wherein each R¹ is ethyl. According to a preferred embodiment, the present disclosure relates to a compound or pharmaceutically acceptable salt thereof of formula (IV), wherein each R¹ is i-propyl. According to a preferred embodiment, the present disclosure relates to a compound or pharmaceutically acceptable salt thereof of formula (IV), wherein each R¹ is -CH₂-O-CO-O-C(CH₃)₃.

According to a further preferred embodiment, the present disclosure relates to a compound or pharmaceutically acceptable salt thereof of formula (IVa)

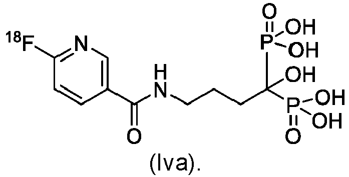

According to a further preferred embodiment, the present disclosure relates to a compound or pharmaceutically acceptable salt thereof of formula (IVb) 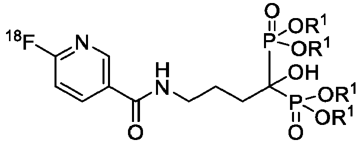 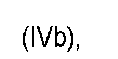 wherein each R¹ is methyl or ethyl, preferably ethyl.

According to one embodiment the present invention relates to a compound of formula (I.a), wherein n is 1, and A is -NR².

According to a preferred embodiment, the present disclosure relates to a compound or pharmaceutically acceptable salt thereof of formula (VI) 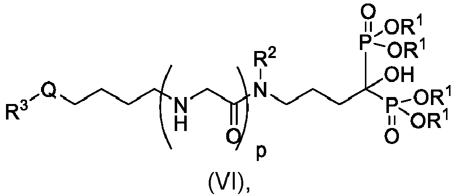 wherein Q is-NH, -NR^{Q}, -N⁺R^{Q}₂X⁻, or -O; wherein X⁻ is a pharmaceutically acceptable anion, preferably X⁻ is F⁻, Cl⁻, Br, I⁻, OH⁻, tosylate, mesylate, triflate, trifluoroacetate, sulfate, acetate, tartrate, benzoate, oxylate, salicylate, or camphorsulfonate; more preferably Br⁻; and wherein R^{Q} is linear or branched (C₁-C₆)alkyl; and wherein p is 0 or 1; and wherein R³ is 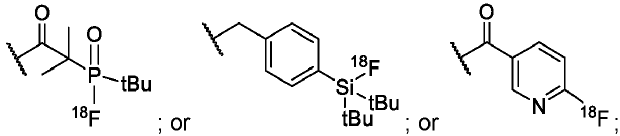 and wherein R² is hydrogen or linear or branched (C₁-C₆)alkyl, preferably R² is hydrogen or methyl; and wherein each R¹ is independently selected from the group consisting of hydrogen, linear or branched (C₁-C₆)alkyl, and -CH₂-O-CO-O-(C₁-C₆)alkyl, preferably wherein each R¹ is hydrogen, methyl, ethyl, i-propyl, or -O(CO)-C(CH₃)₃, more preferably each R¹ is hydrogen, or methyl, most preferably each R¹ is hydrogen.

According to another preferred embodiment, the present disclosure relates to a compound or pharmaceutically acceptable salt thereof of formula (VI), wherein Q is-NH. According to another preferred embodiment, the present disclosure relates to a compound or pharmaceutically acceptable salt thereof of formula (VI), wherein Q is -O. According to another preferred embodiment, the present disclosure relates to a compound or pharmaceutically acceptable salt thereof of formula (VI), wherein Q is -NR^{Q}, wherein R^{Q} is linear or branched (C₁-C₆)alkyl, preferably methyl. According to another preferred embodiment, the present disclosure relates to a compound or pharmaceutically acceptable salt thereof of formula (VI), wherein Q is -NR^{Q}₂X⁻, wherein R^{Q} is linear or branched (C₁-C₆)alkyl, preferably methyl, an wherein X⁻ is F⁻, Cl⁻, Br, I⁻, or OH⁻, tosylate, mesylate, triflate, trifluoroacetate, sulfate, acetate, tartrate, benzoate, oxylate, salicylate, or camphorsulfonate.

According to another preferred embodiment, the present disclosure relates to a compound or pharmaceutically acceptable salt thereof of formula (VI), wherein Q is -N⁺R^{Q}₂X⁻, wherein R^{Q} is linear or branched (C₁-C₆)alkyl, preferably methyl, an wherein X⁻ is F⁻, Cl⁻, Br⁻, I⁻, or OH⁻. According to a preferred embodiment, the present disclosure relates to a compound or pharmaceutically acceptable salt thereof of formula (VI), wherein X⁻ is F⁻. According to another preferred embodiment, the present disclosure relates to a compound or pharmaceutically acceptable salt thereof of formula (VI), wherein X⁻ is Cl⁻. According to another preferred embodiment, the present disclosure relates to a compound or pharmaceutically acceptable salt thereof of formula (VI), wherein X⁻ is Br. According to another preferred embodiment, the present disclosure relates to a compound or pharmaceutically acceptable salt thereof of formula (VI), wherein X⁻ is I⁻. According to another preferred embodiment, the present disclosure relates to a compound or pharmaceutically acceptable salt thereof of formula (VI), wherein X⁻ is OH⁻.

According to a preferred embodiment, the present disclosure relates to a compound or pharmaceutically acceptable salt thereof of formula (VI) p is 0. According to another preferred embodiment, the present disclosure relates to a compound or pharmaceutically acceptable salt thereof of formula (VI) p is 1.

According to a preferred embodiment, the present disclosure relates to a compound or pharmaceutically acceptable salt thereof of formula (VI), wherein R² is hydrogen or linear or branched (C₁-C₆)alkyl. According to another preferred embodiment, the present disclosure relates to a compound or pharmaceutically acceptable salt thereof of formula (VI), wherein R² is hydrogen or methyl.

According to a preferred embodiment, the present disclosure relates to a compound or pharmaceutically acceptable salt thereof of formula (VI), wherein R³ is 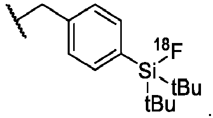 According to a preferred embodiment, the present disclosure relates to a compound or pharmaceutically acceptable salt thereof of formula (VI), wherein R³ is 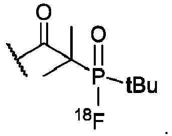 According to a preferred embodiment, the present disclosure relates to a compound or pharmaceutically acceptable salt thereof of formula (VI), wherein R³ is

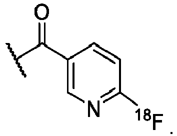

According to a preferred embodiment, the present disclosure relates to a compound or pharmaceutically acceptable salt thereof of formula (VI), wherein each R¹ is hydrogen, methyl, ethyl, i-propyl, or -CH₂-O-CO-O-C(CH₃)₃. According to another preferred embodiment, the present disclosure relates to a compound or pharmaceutically acceptable salt thereof of formula (VI), wherein each R¹ is hydrogen, methyl, ethyl, or i-propyl. According to another preferred embodiment, the present disclosure relates to a compound or pharmaceutically acceptable salt thereof of formula (VI), wherein each R¹ is hydrogen, methyl, or i-propyl. According to another preferred embodiment, the present disclosure relates to a compound or pharmaceutically acceptable salt thereof of formula (VI), wherein each R¹ is hydrogen. According to another preferred embodiment, the present disclosure relates to a compound or pharmaceutically acceptable salt thereof of formula (VI), wherein each R¹ is methyl. According to a preferred embodiment, the present disclosure relates to a compound or pharmaceutically acceptable salt thereof of formula (VI), wherein each R¹ is ethyl. According to another preferred embodiment, the present disclosure relates to a compound or pharmaceutically acceptable salt thereof of formula (VI), wherein each R¹ is i-propyl. According to another preferred embodiment, the present disclosure relates to a compound or pharmaceutically acceptable salt thereof of formula (VI), wherein each R¹ is -CH₂-O-CO-O-C(CH₃)₃.

According to a further preferred embodiment, the present disclosure relates to a compound or pharmaceutically acceptable salt thereof of formula (VIa)

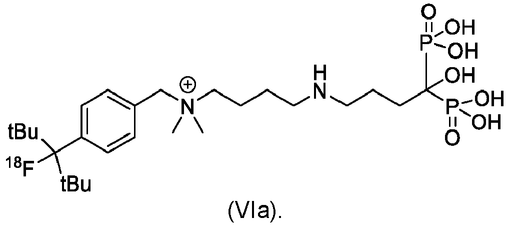

According to a further preferred embodiment, the present disclosure relates to a compound or pharmaceutically acceptable salt thereof of formula (VIb)

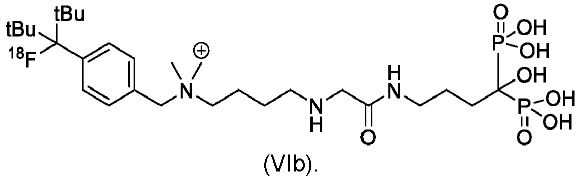

According to a further preferred embodiment, the present disclosure relates to a compound or pharmaceutically acceptable salt thereof of formula (VIc)

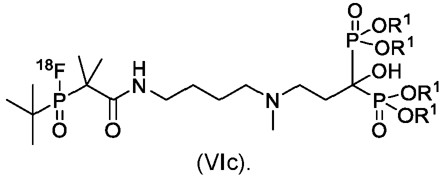

### Derivatives of zolendronic acid

According to one embodiment the present invention relates to a derivative of zolendronic acid. In other words, according to one embodiment the present invention relates to a compound of formula (I.a), wherein n is 2, A is 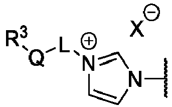 , and wherein X⁻ is a pharmaceutically acceptable anion, preferably X⁻ is F⁻, Cl⁻, Br, I⁻, or OH⁻; more preferably Br⁻, and wherein L and Q are absent. According to a further preferred embodiment, the present disclosure relates to a compound or pharmaceutically acceptable salt thereof of formula (V)

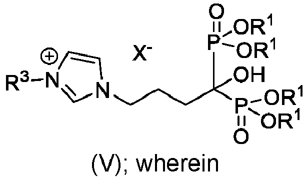

X⁻ is a pharmaceutically acceptable anion, preferably X⁻ is F⁻, Cl⁻, Br⁻, I⁻, or OH⁻; more preferably Br⁻; and wherein R³ is 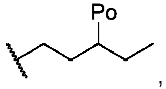 wherein Po is ¹⁸F or O¹¹CH₃, preferably ¹⁸F; or 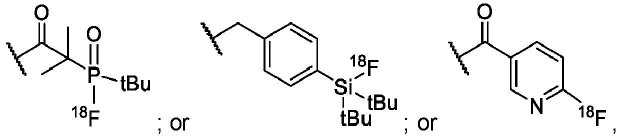 preferably wherein R³ is 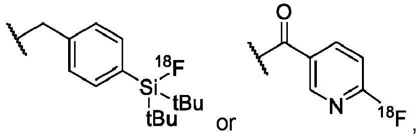 more preferably R³ is 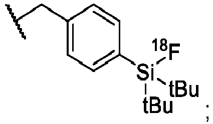 and wherein each R¹ is independently selected from the group consisting of hydrogen, linear or branched (C₁-C₆)alkyl, and -CH₂-O-CO-O-(C₁-C₆)alkyl, preferably wherein each R¹ is hydrogen, methyl, ethyl, or i-propyl, more preferably each R¹ is hydrogen, methyl, or i-propyl, most preferably each R¹ is hydrogen or methyl.

According to a further preferred embodiment, the present disclosure relates to a compound or pharmaceutically acceptable salt thereof of formula (V)

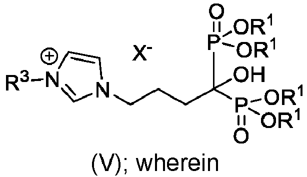

X⁻ is a pharmaceutically acceptable anion, preferably X⁻ is F⁻, Cl⁻, Br⁻, I⁻, or OH⁻; more preferably Br⁻; and wherein R³ is R³ is a group comprising the positron emitting radionuclide Po; and wherein each R¹ is independently selected from the group consisting of hydrogen, linear or branched (C₁-C₆)alkyl,
and -CH₂-O-CO-O-(C₁-C₆)alkyl, preferably wherein each R¹ is hydrogen, methyl, ethyl, or i-propyl, more preferably each R¹ is hydrogen, methyl, or i-propyl, most preferably each R¹ is hydrogen or methyl.

According to a preferred embodiment, the present disclosure relates to a compound or pharmaceutically acceptable salt thereof of formula (V), wherein R³ is 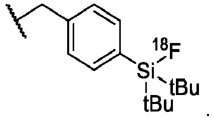 According to a preferred embodiment, the present disclosure relates to a compound or pharmaceutically acceptable salt thereof of formula (V), wherein R³ is 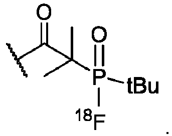 According to a preferred embodiment, the present disclosure relates to a compound or pharmaceutically acceptable salt thereof of formula (V), wherein R³ is

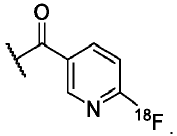

According to a preferred embodiment, the present disclosure relates to a compound or pharmaceutically acceptable salt thereof of formula (V), wherein X⁻ is F⁻. According to another preferred embodiment, the present disclosure relates to a compound or pharmaceutically acceptable salt thereof of formula (V), wherein X⁻ is Cl⁻. According to another preferred embodiment, the present disclosure relates to a compound or pharmaceutically acceptable salt thereof of formula (V), wherein X⁻ is Br⁻. According to another preferred embodiment, the present disclosure relates to a compound or pharmaceutically acceptable salt thereof of formula (V), wherein X⁻ is I⁻. According to another preferred embodiment, the present disclosure relates to a compound or pharmaceutically acceptable salt thereof of formula (V), wherein X⁻ is OH⁻.

According to a preferred embodiment, the present disclosure relates to a compound or pharmaceutically acceptable salt thereof of formula (V), wherein each R¹ is hydrogen, methyl, ethyl, i-propyl, or -CH₂-O-CO-O-C(CH₃)₃. According to another preferred embodiment, the present disclosure relates to a compound or pharmaceutically acceptable salt thereof of formula (V), wherein each R¹ is hydrogen, methyl, ethyl, or i-propyl. According to another preferred embodiment, the present disclosure relates to a compound or pharmaceutically acceptable salt thereof of formula (V), wherein each R¹ is hydrogen, methyl, or i-propyl. According to another preferred embodiment, the present disclosure relates to a compound or pharmaceutically acceptable salt thereof of formula (V), wherein each R¹ is hydrogen. According to another preferred embodiment, the present disclosure relates to a compound or pharmaceutically acceptable salt thereof of formula (V), wherein each R¹ is methyl. According to a preferred embodiment, the present disclosure relates to a compound or pharmaceutically acceptable salt thereof of formula (V), wherein each R¹ is ethyl. According to another preferred embodiment, the present disclosure relates to a compound or pharmaceutically acceptable salt thereof of formula (V), wherein each R¹ is i-propyl. According to another preferred embodiment, the present disclosure relates to a compound or pharmaceutically acceptable salt thereof of formula (V), wherein each R¹ is -CH₂-O-CO-O-C(CH₃)₃.

According to a further preferred embodiment, the present disclosure relates to a compound or pharmaceutically acceptable salt thereof of formula (Va) 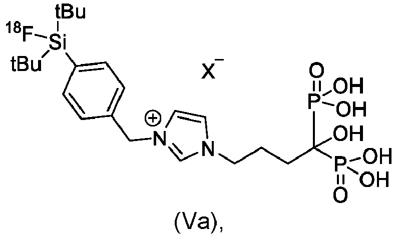 wherein X⁻ is a pharmaceutically acceptable anion, preferably X⁻ is F⁻, Cl⁻, Br, I⁻, or OH⁻; more preferably Br⁻.

According to one embodiment the present invention relates to a compound of formula (I.a), wherein n is 2, A is 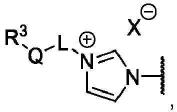 and wherein X⁻ is a pharmaceutically acceptable anion, preferably X⁻ is F⁻, Cl⁻, Br⁻, I⁻, or OH⁻; more preferably Br⁻.

According to a further preferred embodiment, the present disclosure relates to a compound or pharmaceutically acceptable salt thereof of formula (VII) 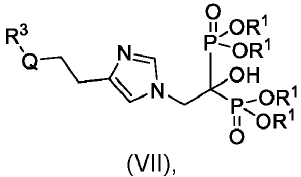 wherein Q is -NH, -NR^{Q}, -N⁺R^{Q}₂X⁻, *N,N*-substituted piperazine or -O; and wherein X⁻ is a pharmaceutically acceptable anion, preferably X⁻ is F⁻, Cl⁻, Br⁻, I⁻, or OH⁻; more preferably Br⁻; and wherein R^{Q} is each independently linear or branched (C₁-C₆)alkyl, preferably each R^{Q} is methyl; preferably Q is -NH; and wherein R³ is 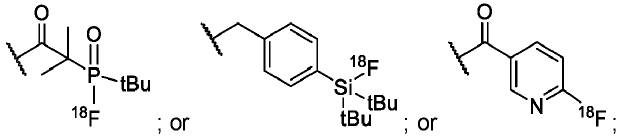 and wherein each R¹ is independently selected from the group consisting of hydrogen, linear or branched (C₁-C₆)alkyl, and -CH₂-O-CO-O-(C₁-C₆)alkyl, preferably wherein each R¹ is hydrogen, methyl, ethyl, i-propyl, or -O(CO)-C(CH₃)₃, more preferably each R¹ is hydrogen, or methyl, most preferably each R¹ is hydrogen.

According to a preferred embodiment, the present disclosure relates to a compound or pharmaceutically acceptable salt thereof of formula (VII), wherein Q is -NH. According to another preferred embodiment, the present disclosure relates to a compound or pharmaceutically acceptable salt thereof of formula (VII), wherein Q is -O. According to another preferred embodiment, the present disclosure relates to a compound or pharmaceutically acceptable salt thereof of formula (VII), wherein Q is *N*,*N*-substituted piperazine. According to another preferred embodiment, the present disclosure relates to a compound or pharmaceutically acceptable salt thereof of formula (VII), wherein Q is -NR^{Q}, wherein R^{Q} is linear or branched (C₁-C₆)alkyl, preferably methyl. According to another preferred embodiment, the present disclosure relates to a compound or pharmaceutically acceptable salt thereof of formula (VII), wherein Q is -N⁺R^{Q}₂X⁻, wherein R^{Q} is linear or branched (C₁-C₆)alkyl, preferably methyl, and wherein X⁻ is F⁻, Cl⁻, Br, I⁻, or OH⁻, tosylate, mesylate, triflate, trifluoroacetate, sulfate, acetate, tartrate, benzoate, oxylate, salicylate, or camphorsulfonate.

According to another preferred embodiment, the present disclosure relates to a compound or pharmaceutically acceptable salt thereof of formula (VII), wherein Q is -N⁺R^{Q}₂X⁻, wherein R^{Q} is linear or branched (C₁-C₆)alkyl, preferably methyl, and wherein X⁻ is F⁻, Cl⁻, Br⁻, I⁻, or OH⁻. According to a preferred embodiment, the present disclosure relates to a compound or pharmaceutically acceptable salt thereof of formula (VII), wherein X⁻ is F⁻. According to another preferred embodiment, the present disclosure relates to a compound or pharmaceutically acceptable salt thereof of formula (VII), wherein X⁻ is Cl⁻. According to another preferred embodiment, the present disclosure relates to a compound or pharmaceutically acceptable salt thereof of formula (VII), wherein X⁻ is Br⁻. According to another preferred embodiment, the present disclosure relates to a compound or pharmaceutically acceptable salt thereof of formula (VII), wherein X⁻ is I⁻. According to another preferred embodiment, the present disclosure relates to a compound or pharmaceutically acceptable salt thereof of formula (VII), wherein X⁻ is OH⁻.

According to a preferred embodiment, the present disclosure relates to a compound or pharmaceutically acceptable salt thereof of formula (VII), wherein R³ is 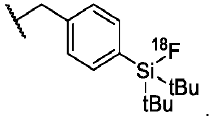 According to a preferred embodiment, the present disclosure relates to a compound or pharmaceutically acceptable salt thereof of formula (VII), wherein R³ is 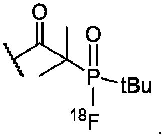 According to a preferred embodiment, the present disclosure relates to a compound or pharmaceutically acceptable salt thereof of formula (VII), wherein R³ is

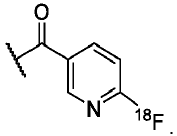

According to a preferred embodiment, the present disclosure relates to a compound or pharmaceutically acceptable salt thereof of formula (VII), wherein each R¹ is hydrogen, methyl, ethyl, i-propyl, or -CH₂-O-CO-O-C(CH₃)₃. According to another preferred embodiment, the present disclosure relates to a compound or pharmaceutically acceptable salt thereof of formula (VII), wherein each R¹ is hydrogen, methyl, ethyl, or i-propyl. According to another preferred embodiment, the present disclosure relates to a compound or pharmaceutically acceptable salt thereof of formula (VII), wherein each R¹ is hydrogen, methyl, or i-propyl. According to another preferred embodiment, the present disclosure relates to a compound or pharmaceutically acceptable salt thereof of formula (VII), wherein each R¹ is hydrogen. According to another preferred embodiment, the present disclosure relates to a compound or pharmaceutically acceptable salt thereof of formula (VII), wherein each R¹ is methyl. According to a preferred embodiment, the present disclosure relates to a compound or pharmaceutically acceptable salt thereof of formula (VII), wherein each R¹ is ethyl. According to another preferred embodiment, the present disclosure relates to a compound or pharmaceutically acceptable salt thereof of formula (VII), wherein each R¹ is i-propyl. According to another preferred embodiment, the present disclosure relates to a compound or pharmaceutically acceptable salt thereof of formula (VII), wherein each R¹ is -CH₂-O-CO-O-C(CH₃)₃.

According to a further preferred embodiment, the present disclosure relates to a compound or pharmaceutically acceptable salt thereof of formula (VIIa)

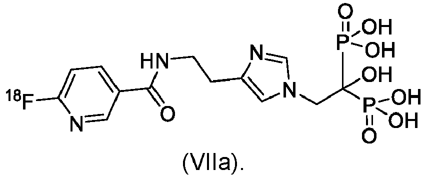

### Derivatives of risedronic acid

According to one embodiment the present invention relates to a derivative of risedronic acid. In other words, according to one embodiment the present invention relates to a compound of formula (I.a), wherein n is 0, and R³-Q-L-A- is 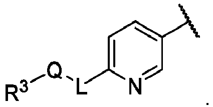 According to a preferred embodiment, the present disclosure relates to a compound or pharmaceutically acceptable salt thereof of formula (VIII) 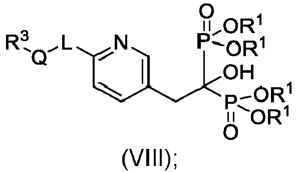 wherein L and Q are absent; or wherein L is a divalent linker group; and wherein Q is -NH, -NR^{Q}, -N⁺R^{Q}₂X⁻, -O, *N*,*N*-substituted piperazine, or 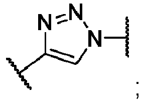 and wherein X⁻ is a pharmaceutically acceptable anion, preferably X⁻ is F⁻, Cl⁻, Br, I⁻, OH⁻, tosylate, mesylate, triflate, trifluoroacetate, sulfate, acetate, tartrate, benzoate, oxylate, salicylate, or camphorsulfonate; more preferably Br⁻; and wherein R^{Q} is each independently linear or branched (C₁-C₆)alkyl, preferably each R^{Q} is methyl; and wherein R³ is a group comprising the positron emitting radionuclide Po; and wherein each R¹ is independently selected from the group consisting of hydrogen, linear or branched (C₁-C₆)alkyl, and -CH₂-O-CO-O-(C₁-C₆)alkyl, preferably wherein each R¹ is hydrogen, methyl, ethyl, or i-propyl, more preferably each R¹ is hydrogen, methyl, or i-propyl, most preferably each R¹ is hydrogen or methyl.

According to a preferred embodiment, the present disclosure relates to a compound or pharmaceutically acceptable salt thereof of formula (VIII), wherein R³ is 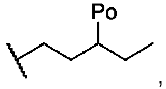 wherein Po is ¹⁸F or O¹¹CH₃, preferably ¹⁸F; or 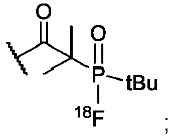 or 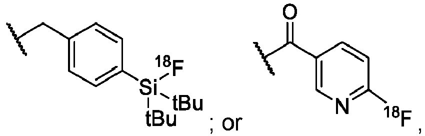 preferably wherein R³ is 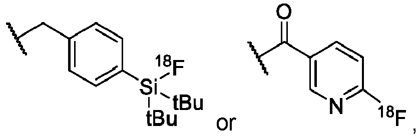 more preferably R³ is

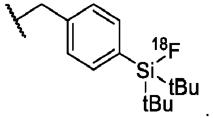

According to a preferred embodiment, the present disclosure relates to a compound or pharmaceutically acceptable salt thereof of formula (VIII), wherein R³ is 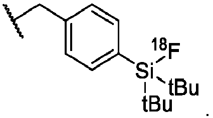 According to a preferred embodiment, the present disclosure relates to a compound or pharmaceutically acceptable salt thereof of formula (VIII), wherein R³ is 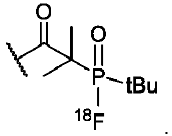 According to a preferred embodiment, the present disclosure relates to a compound or pharmaceutically acceptable salt thereof of formula (VIII), wherein R³ is

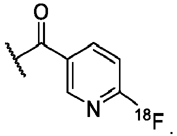

According to a preferred embodiment, the present disclosure relates to a compound or pharmaceutically acceptable salt thereof of formula (VIII), wherein Q is -NH. According to another preferred embodiment, the present disclosure relates to a compound or pharmaceutically acceptable salt thereof of formula (VIII), wherein Q is -O. According to another preferred embodiment, the present disclosure relates to a compound or pharmaceutically acceptable salt thereof of formula (VIII), wherein Q is *N*,*N*-substituted piperazine. According to another preferred embodiment, the present disclosure relates to a compound or pharmaceutically acceptable salt thereof of formula (VIII), wherein Q is -NR^{Q}, wherein R^{Q} is linear or branched (C₁-C₆)alkyl, preferably methyl. According to another preferred embodiment, the present disclosure relates to a compound or pharmaceutically acceptable salt thereof of formula (VIII), wherein Q is -N⁺R^{Q}₂X⁻, wherein R^{Q} is linear or branched (C₁-C₆)alkyl, preferably methyl, and wherein X⁻ is F⁻, Cl⁻, Br, I⁻, or OH⁻, tosylate, mesylate, triflate, trifluoroacetate, sulfate, acetate, tartrate, benzoate, oxylate, salicylate, or camphorsulfonate.

According to a preferred embodiment, the present disclosure relates to a compound or pharmaceutically acceptable salt thereof of formula (VIII), wherein each R¹ is hydrogen, methyl, ethyl, i-propyl, or -CH₂-O-CO-O-C(CH₃)₃. According to another preferred embodiment, the present disclosure relates to a compound or pharmaceutically acceptable salt thereof of formula (VIII), wherein each R¹ is hydrogen, methyl, ethyl, or i-propyl. According to another preferred embodiment, the present disclosure relates to a compound or pharmaceutically acceptable salt thereof of formula (VIII), wherein each R¹ is hydrogen, methyl, or i-propyl. According to another preferred embodiment, the present disclosure relates to a compound or pharmaceutically acceptable salt thereof of formula (VIII), wherein each R¹ is hydrogen. According to another preferred embodiment, the present disclosure relates to a compound or pharmaceutically acceptable salt thereof of formula (VIII), wherein each R¹ is methyl. According to a preferred embodiment, the present disclosure relates to a compound or pharmaceutically acceptable salt thereof of formula (VIII), wherein each R¹ is ethyl. According to another preferred embodiment, the present disclosure relates to a compound or pharmaceutically acceptable salt thereof of formula (VIII), wherein each R¹ is i-propyl. According to another preferred embodiment, the present disclosure relates to a compound or pharmaceutically acceptable salt thereof of formula (VIII), wherein each R¹ is -CH₂-O-CO-O-C(CH₃)₃.

According to a further preferred embodiment, the present disclosure relates to a compound or pharmaceutically acceptable salt thereof of formula (Villa)

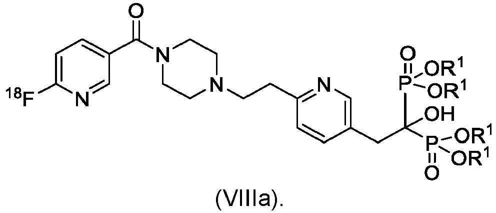

According to another preferred embodiment the present invention relates to a compound of formula (I.a), wherein n is 0, and R³-Q-L-A- is 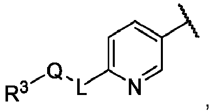 wherein L-Q is -CH₂-CH₂-NH-. In other words, the present disclosure relates to a compound or pharmaceutically acceptable salt thereof of formula (IX) 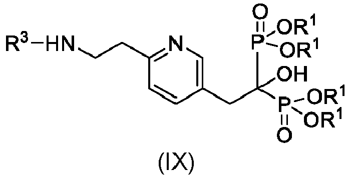 wherein R³ is a group comprising the positron emitting radionuclide Po; and wherein each R¹ is independently selected from the group consisting of hydrogen, linear or branched (C₁-C₆)alkyl, and -CH₂-O-CO-O-(C₁-C₆)alkyl, preferably wherein each R¹ is hydrogen, methyl, ethyl, or i-propyl, more preferably each R¹ is hydrogen, methyl, or i-propyl, most preferably each R¹ is hydrogen or methyl.

According to a preferred embodiment, the present disclosure relates to a compound or pharmaceutically acceptable salt thereof of formula (IX), wherein R³ is 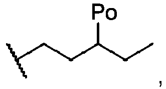 wherein Po is ¹⁸F or O¹¹CH₃, preferably ¹⁸F; or 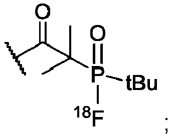 or 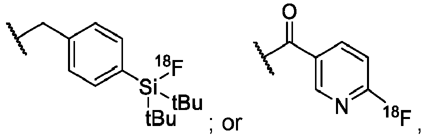 preferably wherein R³ is 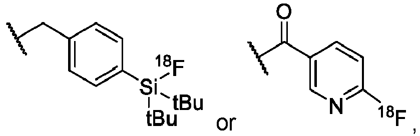 more preferably R³ is

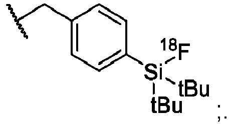

According to a preferred embodiment, the present disclosure relates to a compound or pharmaceutically acceptable salt thereof of formula (IX), wherein R³ is 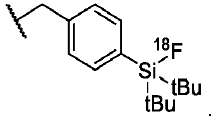 According to a preferred embodiment, the present disclosure relates to a compound or pharmaceutically acceptable salt thereof of formula (IX), wherein R³ is 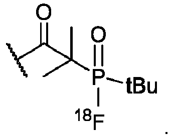 According to a preferred embodiment, the present disclosure relates to a compound or pharmaceutically acceptable salt thereof of formula (IX), wherein R³ is

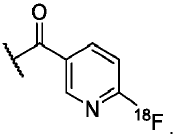

According to a preferred embodiment, the present disclosure relates to a compound or pharmaceutically acceptable salt thereof of formula (IX), wherein each R¹ is hydrogen, methyl, ethyl, i-propyl, or -CH₂-O-CO-O-C(CH₃)₃. According to another preferred embodiment, the present disclosure relates to a compound or pharmaceutically acceptable salt thereof of formula (IX), wherein each R¹ is hydrogen, methyl, ethyl, or i-propyl. According to another preferred embodiment, the present disclosure relates to a compound or pharmaceutically acceptable salt thereof of formula (IX), wherein each R¹ is hydrogen, methyl, or i-propyl. According to another preferred embodiment, the present disclosure relates to a compound or pharmaceutically acceptable salt thereof of formula (IX), wherein each R¹ is hydrogen. According to another preferred embodiment, the present disclosure relates to a compound or pharmaceutically acceptable salt thereof of formula (IX), wherein each R¹ is methyl. According to a preferred embodiment, the present disclosure relates to a compound or pharmaceutically acceptable salt thereof of formula (IX), wherein each R¹ is ethyl. According to another preferred embodiment, the present disclosure relates to a compound or pharmaceutically acceptable salt thereof of formula (IX), wherein each R¹ is i-propyl. According to another preferred embodiment, the present disclosure relates to a compound or pharmaceutically acceptable salt thereof of formula (IX), wherein each R¹ is -CH₂-O-CO-O-C(CH₃)₃.

According to a further preferred embodiment, the present disclosure relates to a compound or pharmaceutically acceptable salt thereof of formula (IXa)

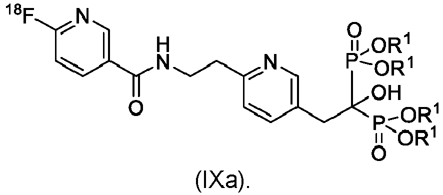

### Medical use

In a further aspect, the present disclosure relates to a compound or pharmaceutically acceptable salt thereof according to the present disclosure for use in medicine.

In a further aspect, the present disclosure relates to a compound or pharmaceutically acceptable salt thereof according to the present disclosure for use in a method of diagnosis.

In a further aspect, the present disclosure relates to a compound or pharmaceutically acceptable salt thereof according to the present disclosure for use in a method of diagnosis of amyloidosis.

In a further aspect, the present disclosure relates to a compound or pharmaceutically acceptable salt thereof according to the present disclosure for use in a method of diagnosis of cardiac amyloidosis.

### Method of manufacture

According to a further aspect, the present disclosure relates to a first method of preparation of a compound according to the present disclosure, the method comprising the steps of
a) providing a compound of formula (Ia) 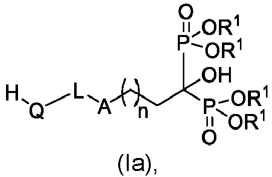 wherein each R¹ is independently hydrogen, a pharmaceutically acceptable cation, or a protection group; and wherein n is an integer selected from the group consisting of 0, 1, 2, and 3; and wherein A is -NR², or (4 to 8
   membered)heterocyclyl, or (4 to 8 membered)cyclyl, or squaramide, or (5 to 6 membered)heteroaryl, or phenyl; and wherein R² is hydrogen or linear or branched (C₁-C₆)alkyl; and wherein L and Q are absent; or wherein L is a divalent linker group, and wherein Q is -NH, -NR^{Q}, -N⁺R^{Q}₂X⁻, -O, or *N*,*N*-substituted piperazine; wherein R^{Q} is each independently linear or branched (C₁-C₆)alkyl, preferably each R^{Q} is methyl; and
b) providing a compound of formula R³-Y, wherein R³-Y is 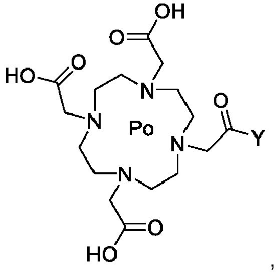 wherein Po is ⁶⁴Cu or ⁶⁸Ga, preferably ⁶⁸Ga; or 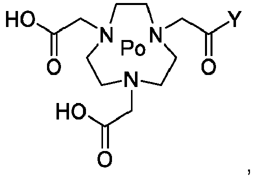 wherein Po is ⁶⁴Cu or ⁶⁸Ga, preferably ⁶⁸Ga; or 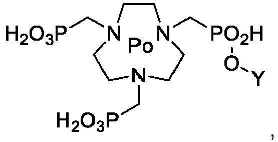 wherein Po is ⁶⁴Cu or ⁶⁸Ga, preferably ⁶⁸Ga; or 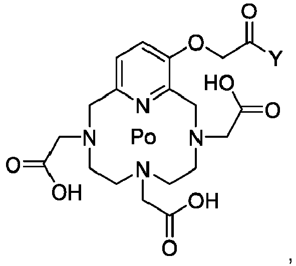 wherein Po is ⁶⁴Cu or ⁶⁸Ga, preferably ⁶⁸Ga; or 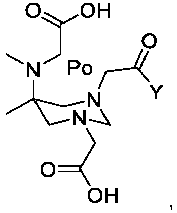 wherein Po is ⁶⁴Cu or ⁶⁸Ga, preferably ⁶⁸Ga; or 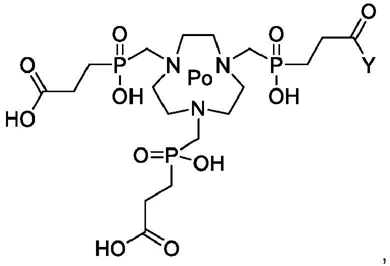 wherein Po is ⁶⁴Cu or ⁶⁸Ga, preferably ⁶⁸Ga; or 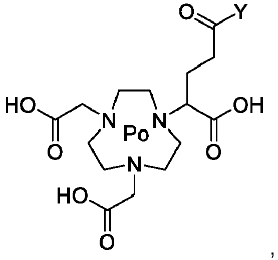 wherein Po is ⁶⁴Cu or ⁶⁸Ga, preferably ⁶⁸Ga; or 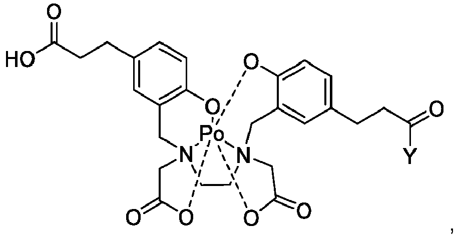 wherein Po is ⁶⁴Cu or ⁶⁸Ga, preferably ⁶⁸Ga; or 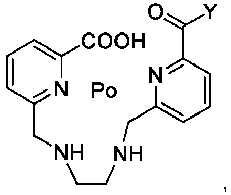 wherein Po is ⁶⁴Cu or ⁶⁸Ga, preferably ⁶⁸Ga; or 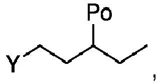 wherein Po is ¹⁸F or -O¹¹CH₃, preferably ¹⁸F; or Y-¹¹CH₃, or HO-¹¹CH₃; 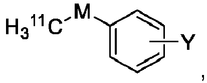 wherein M is O, or -NH, or CH₂O; or 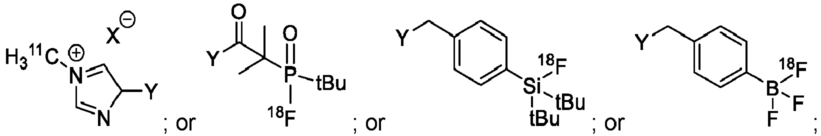 or 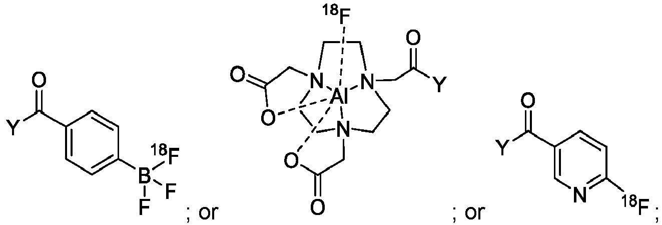 and wherein Y is an electron withdrawing group, preferably Y is selected from the group consisting of -Cl, -Br, -O-C₆F₅H, -O(CO)-(C₁-C₆)alkyl, and -OH; and
c) reacting the compounds of formula (Ia) and any one of R³-Y at a temperature of from about 60 °C to about 140 °C, wherein when Y is -OH, step b) further comprises providing dry DMF and a coupling agent selected from the group consisting of EDC, HATU, HOBt, HBTU, and HATU.

According to a preferred embodiment, the present disclosure relates to a first method of preparation of a compound according to the present disclosure, wherein step c) is performed for a period of from about 1 min to about 60 min.

According to a preferred embodiment, the present disclosure relates to a first method of preparation of a compound according to the present disclosure, wherein step c) is performed in dry DMF or dry DMSO.

According to another aspect, the present disclosure relates to a second method of preparation of a compound according to the present disclosure the method comprising the steps of
a) providing a compound of formula (Ib) 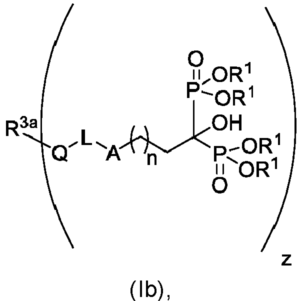 wherein z is 1, 2, or 3, preferably z is 1; and wherein each R¹ is independently hydrogen, a pharmaceutically acceptable cation, or a protection group; and wherein n is an integer selected from the group consisting of 0, 1, 2, and 3; and wherein A is -NR², or (4 to 8 membered)heterocyclyl, or (4 to 8 membered)cyclyl, or squaramide, or (5 to 6 membered)heteroaryl, or phenyl; and wherein R² is hydrogen or linear or branched (C₁-C₆)alkyl; and wherein L and Q are absent; or L is a divalent linker group; and wherein Q is -NH, -NR^{Q}, -N⁺R^{Q}₂X⁻, -O, or *N*,*N*-substituted piperazine; wherein R^{Q} is each independently linear or branched (C₁-C₆)alkyl, preferably each R^{Q} is methyl; and wherein R^{3a} is 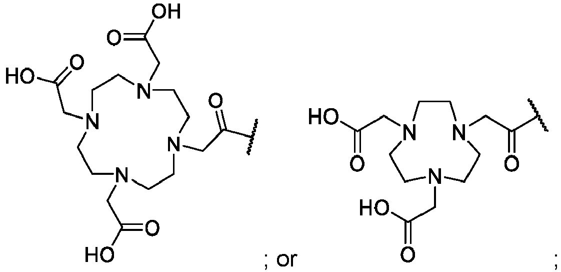 or 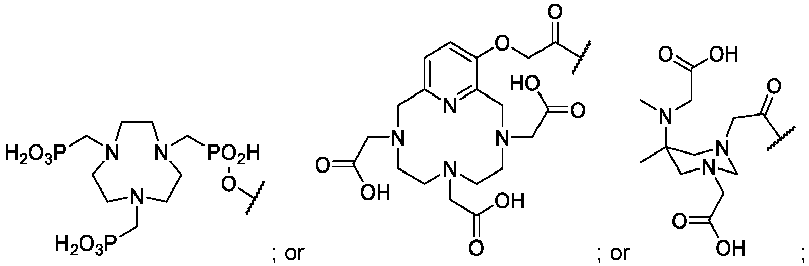 or 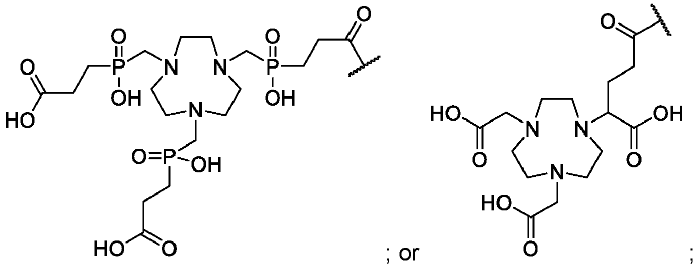 or 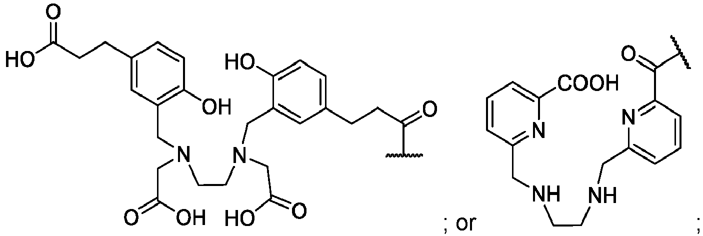 or 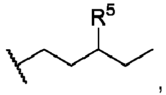 wherein R⁵ is OTs, OMs, OTf, or I; or 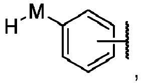 wherein M is O, or -NH, or CH₂O; or 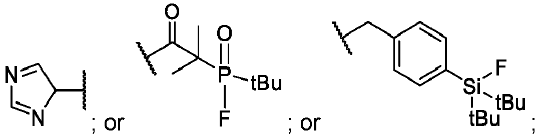 or 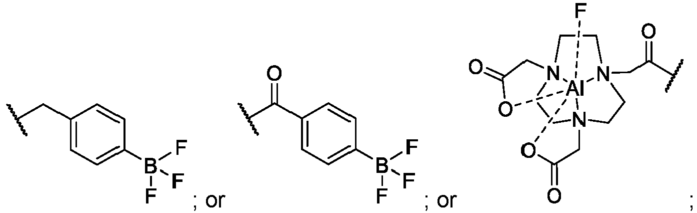 or 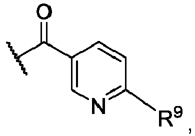 wherein R⁹ is an ammonium salt, or H; and
b) reacting the compound of formula (Ib) with a group Po* comprising th positron emitting radionuclide Po, wherein Po* is -¹¹CH₃, ¹³N, ¹⁵O, ¹⁸F⁻, ⁶⁴Cu or ⁶⁸Ga, preferably -¹¹CH₃, ¹⁸F⁻, and ⁶⁸Ga, more preferably ¹⁸F⁻ or ⁶⁸Ga.

According to a preferred embodiment, the present disclosure relates to a method of preparation of a compound according to the present disclosure, wherein step b) is performed for a period of time of from about 1 min to about 60 min.

According to a preferred embodiment, the present disclosure relates to a second method of preparation of a compound according to the present disclosure, wherein step b) is performed at a temperature of from about 80 °C to about 140 °C.

According to a preferred embodiment, the present disclosure relates to a second method of preparation of a compound according to the present disclosure, wherein step b) is performed in dry DMF or dry DMSO.

According to another aspect, the present disclosure relates to a third method of preparation of a compound according to the present disclosure the method comprising the steps of,
a) providing a compound of formula (Ic) 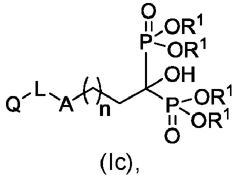 wherein each R¹ is independently hydrogen, a pharmaceutically acceptable cation, or a protection group; and wherein n is an integer selected from the group consisting of 0, 1, 2, and 3; and wherein A is -NR², or (4 to 8 membered)heterocyclyl, or (4 to 8 membered)cyclyl, or squaramide, or (5 to 6 membered)heteroaryl, or phenyl; and wherein R² is hydrogen or linear or branched (C₁-C₆)alkyl; and wherein L is (C₁-C₁₀) alkyl, and Q is -C≡CH; and
b) providing a compound of formula 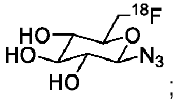 and
c) reacting the compounds of formula (Ic) and step b) at a temperature of from about 60 °C to about 140 °C in the presence of a Cu(I) salt.

According to a preferred embodiment, the present disclosure relates to a third method of preparation of a compound according to the present disclosure, wherein step c) is performed for a period of time of from about 1 min to about 60 min.

According to a preferred embodiment, the present disclosure relates to a third method of preparation of a compound according to the present disclosure, wherein step c) is performed at a temperature of from about 80 °C to about 140 °C.

According to a preferred embodiment, the present disclosure relates to a third method of preparation of a compound according to the present disclosure, wherein step c) is performed in dry DMF or dry DMSO.

### General aspects and definitions

In a further embodiment, the present disclosure relates to a compound or pharmaceutically acceptable salt thereof of formula (I), or formula (II), or formula (III), or formula (IV), or formula (V), or formula (VI), or formula (VII), wherein the compound is not a derivative of risedronic acid.

In a further embodiment, the present disclosure relates to a compound or pharmaceutically acceptable salt thereof of formula (I), or formula (II), or formula (III), or formula (IV), or formula (V), or formula (VI), or formula (VII), wherein the compound is not 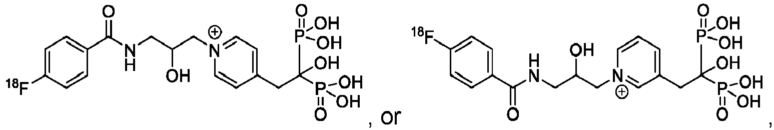 or 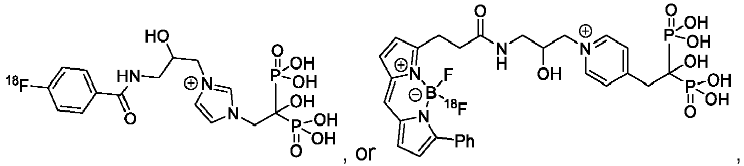 or 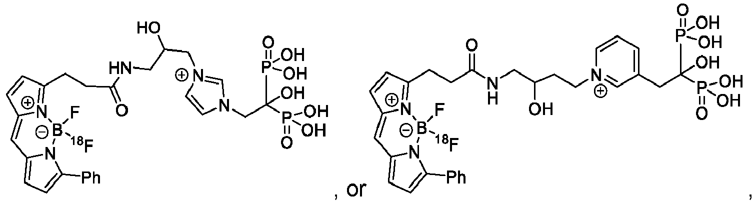 or 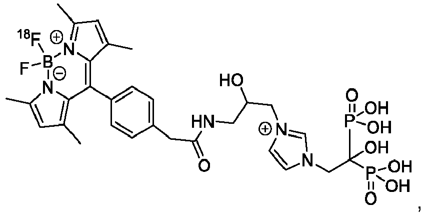 or 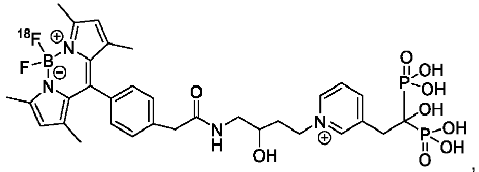 or

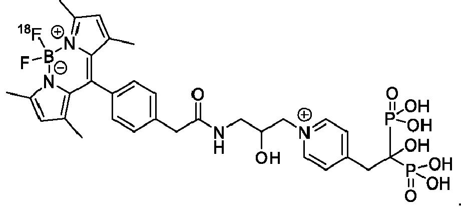

As used herein, the term "pharmaceutically acceptable salts" refers to salts that retain the desired biological activity of the above-identified compounds and include pharmaceutically acceptable acid addition salts and base addition salts. Suitable pharmaceutically acceptable acid addition salts of compounds of the present disclosure may be prepared from an inorganic acid or from an organic acid. Examples of such inorganic acids are hydrochloric, sulfuric, and phosphoric acid. Appropriate organic acids may be selected from aliphatic, cycloaliphatic, aromatic, heterocyclic carboxylic and sulfonic classes of organic acids, examples of which are formic, acetic, propionic, succinic, glycolic, gluconic, lactic, malic, tartaric, citric, fumaric, maleic, alkyl sulfonic, arylsulfonic. Suitable pharmaceutically acceptable base addition salts of compounds of the present disclosure include metallic salts made from lithium, sodium, potassium, aluminium, and zinc, and organic salts made from organic bases such as choline, diethanolamine, morpholine. Other examples of organic salts are: ammonium salts, quaternary salts such as tetramethylammonium salt; amino acid addition salts such as salts with glycine and arginine.

As used herein, the term "pharmaceutically acceptable cation" refers to a cation that is not toxic to mammals. Preferred pharmaceutically acceptable cations are lithium, sodium, potassium, aluminium, and zinc, cations made from organic bases such as choline, diethanolamine, morpholine, ammonium salts, quaternary salts such as tetramethylammonium salt; amino acid addition salts such as salts with glycine and arginine.

As used herein, the term "pharmaceutically acceptable anion" refers to an anion that is not toxic to mammals. Said pharmaceutically acceptable anion may have one, two, three, or more negative charges. Preferred pharmaceutically acceptable anions are F⁻, Cl⁻, Br⁻, I⁻, OH⁻, tosylate, mesylate, triflate, trifluoroacetate, sulfate, acetate, tartrate, benzoate, oxylate, salicylate, or camphorsulfonate. Further preferred pharmaceutically acceptable anions are F⁻, Cl⁻, Br, I⁻, or OH⁻. A particularly preferred pharmaceutically acceptable anion is Br.

A "protection group" as used herein, refers to a group that can be selectively cleaved of a bisphosphonate under acidic or basic conditions or a group that can be selectively cleaved of a bisphosphonate metabolically in the body of a mammal. A preferred protection group is methyl, ethyl, n-propyl, i-propyl, n-butyl, i-butyl, t-butyl, -CH₂-O(CO)-CH₃, -CH₂-O(CO)-CH₂CH₃, -CH₂-O(CO)-CH(CH₃)₂, -CH₂-O(CO)-C(CH₃)₃, and 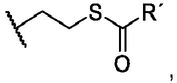 wherein R' is methy or ethyl. Other preferred protection groups are attached to two functional groups form a structure of 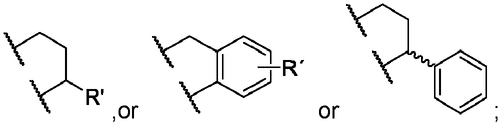 wherein R' is hydrogen or (C₁-C₁₀)alkyl.

Further suitable protection group can be found in Heidel, H.-K.; et al. Phosphonate prodrugs: an overview and recent advances. Future Med. Chem. 2019, 11, 1625-1643, which is incorporated herein by reference.

As used herein, the term "(4 to 8 membered)heterocyclyl" refers to a saturated or partially saturated monocyclic or bicyclic ring containing of from 4 to 8 atoms, wherein at least one of said 4 to 8 atoms is a heteroatom selected from nitrogen, sulfur, or oxygen, preferably said "(4 to 8 membered)heterocyclyl exhibits from 1 to 3 heteroatoms. Preferred (4 to 8 membered)heterocyclyl groups are selected from pyrrolidyl, tetrahydrofuryl, tetrahydrothiofuranyl, piperidyl, piperazyl, tetrahydropyranyl, morphilino, 1,3-diazapane, 1,4-diazapane, 1,4-oxazepane, and 1,4-oxathiapane. The group may be a terminal group or a bridging group. Further (4 to 8 membered)heterocyclyl groups include: 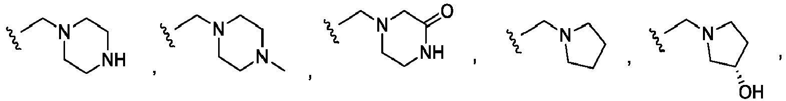 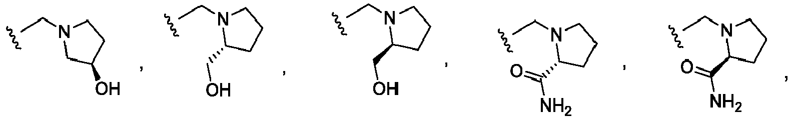 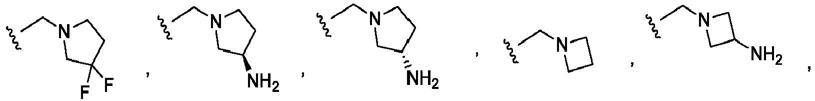 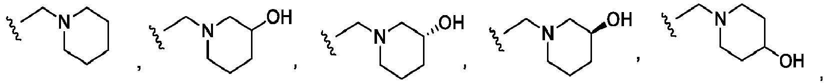 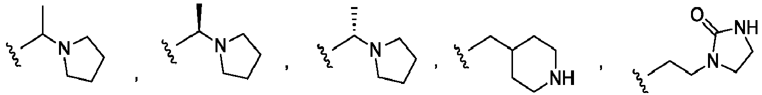 and

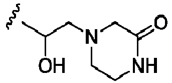

As used herein reference to the normal chain when used in the context of a bridging group refers to the direct chain of atoms linking the two terminal positions of the bridging group

For the purpose of the present disclosure, the term "(4 to 8 membered) cyclyl" as used by itself or as part of another group refers to saturated and partially unsaturated (e.g. containing one or two double bonds) cyclic aliphatic hydrocarbons containing one or two rings having from four to eight carbon atoms (*i.e.,* C₄-C₈ cycloalkyl) or the number of carbons designated. In one embodiment, the (4 to 8 membered) cyclyl group has two rings. In one embodiment, the (4 to 8 membered) cyclyl group has one ring. In one embodiment, the (4 to 8 membered) cyclyl group is a saturated cyclic aliphatic hydrocarbon containing one or two rings, preferably one ring, and having 4, 5, 6, 7, or 8 carbon atoms. In another embodiment, the (4 to 8 membered) cyclyl group is selected as a C₄-C₆ cycloalkyl group. Non-limiting exemplary (4 to 8 membered) cyclyl groups include cyclobutyl, cyclopentyl, cyclohexyl, cycloheptyl, cyclooctyl, and the like. The group may be a terminal group or a bridging group.

For the purpose of the present disclosure, the term "aryl" as used by itself or as part of another group refers to a mono-, bi-, or tricyclic aromatic ring system having from six to fourteen, i.e. 6, 7, 8, 9, 10, 11, 12, 13 or 14, carbon atoms (*i.e.,* C₆-C₁₄ aryl). Non-limiting exemplary aryl groups include phenyl (abbreviated as "Ph"), naphthyl, phenanthryl, anthracyl, indenyl, azulenyl, biphenyl, biphenylenyl, and fluorenyl groups. In one embodiment, the aryl group is chosen from phenyl or naphthyl.

For the purpose of the present disclosure, the term "substituted aryl" as used herein by itself or as part of another group means that the aryl as defined above is substituted with one to ten, preferably one to eight, more preferably one to five substituents independently chosen from halo, nitro, cyano, hydroxy, amino, alkylamino, dialkylamino, haloalkyl, hydroxyalkyl, alkoxy, haloalkoxy, aryloxy, heteroaryloxy, aralkyloxy, alkylthio, carboxamido, sulfonamido, alkylcarbonyl, arylcarbonyl, alkylsulfonyl, arylsulfonyl, ureido, guanidino, carboxy, carboxyalkyl, alkyl, cycloalkyl, alkenyl, alkynyl, aryl, heteroaryl, heterocyclo, alkoxyalkyl, (amino)alkyl, hydroxyalkylamino, (alkylamino)alkyl, (dialkylamino)alkyl, (cyano)alkyl, (carboxamido)alkyl, mercaptoalkyl, (heterocyclo)alkyl, (cycloalkylamino)alkyl, (C₁-C₄ haloalkoxy)alkyl, or (heteroaryl)alkyl. In one embodiment, the substituted aryl is a substituted phenyl. In one embodiment, the substituted phenyl has four substituents. In another embodiment, the substituted phenyl has three substituents. In another embodiment, the substituted phenyl has two substituents. In another embodiment, the substituted phenyl has one substituent. Non-limiting exemplary substituted aryl groups include 2-methylphenyl, 2-methoxyphenyl, 2-fluorophenyl, 2-chlorophenyl, 2-bromophenyl, 3-methylphenyl, 3-methoxyphenyl, 3-fluorophenyl, 3-chlorophenyl, 4-methylphenyl, 4-ethylphenyl, 4-methoxyphenyl, 4-fluorophenyl, 4-chlorophenyl, 2,6-di-fluorophenyl, 2,6-di-chlorophenyl, 2-methyl, 3-methoxyphenyl, 2-ethyl, 3-methoxyphenyl, 3,4-di-methoxyphenyl, 3,5-di-fluorophenyl 3,5-dimethylphenyl, 3,5-dimethoxy, 4-methylphenyl, 2-fluoro-3-chlorophenyl, and 3-chloro-4-fluorophenyl. The term substituted aryl is meant to include groups having fused optionally substituted cycloalkyl and fused optionally substituted heterocyclo rings. Examples include

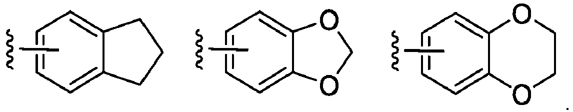

For the purpose of the present disclosure, the term "haloalkyl" as used by itself or as part of another group refers to an alkyl group substituted by one or more fluorine, chlorine, bromine and/or iodine atoms. In one embodiment, the alkyl group is substituted by one, two, or three fluorine and/or chlorine atoms. In another embodiment, the haloalkyl group is chosen from a C₁₋₄ haloalkyl group. Non-limiting exemplary haloalkyl groups include fluoromethyl, difluoromethyl, trifluoromethyl, pentafluoroethyl, 1,1-difluoroethyl, 2,2-difluoroethyl, 2,2,2-trifluoroethyl, 3,3,3-trifluoropropyl, 4,4,4-trifluorobutyl, and trichloromethyl groups.

For the purpose of the present disclosure, the term "(hydroxy)haloalkyl" as used by itself or as part of another group refers to an alkyl group substituted by one or more halogen atoms and one hydroxy group. A non-limiting exemplary (hydroxy)haloalkyl group is -CH(OH)CF₃.

For the purpose of the present disclosure, the term "hydroxyalkyl" as used by itself or as part of another group refers to an alkyl group substituted with one or more, e.g., one, two, or three, hydroxy groups. In one embodiment, the hydroxyalkyl group is a monohydroxyalkyl group, *i.e.,* substituted with one hydroxy group. In another embodiment, the hydroxyalkyl group is a dihydroxyalkyl group, *i.e.,* substituted with two hydroxy groups. In another embodiment, the hydroxyalkyl group is chosen from a C₁₋₄ hydroxyalkyl group. Non-limiting exemplary hydroxyalkyl groups include hydroxymethyl, hydroxyethyl, hydroxypropyl and hydroxybutyl groups, such as 1-hydroxyethyl, 2-hydroxyethyl, 1,2-dihydroxyethyl, 2-hydroxypropyl, 3-hydroxypropyl, 3-hydroxybutyl, 4-hydroxybutyl, 2-hydroxy-1-methylpropyl, and 1,3-dihydroxyprop-2-yl.

For the purpose of the present disclosure, the term "cycloalkyl" as used by itself or as part of another group refers to saturated and partially unsaturated (containing one or two double bonds) cyclic aliphatic hydrocarbons containing one to three rings having from three to twelve carbon atoms (*i.e.,* C₃₋₁₂ cycloalkyl) or the number of carbons designated. In one embodiment, the cycloalkyl group has two rings. In one embodiment, the cycloalkyl group has one ring. In one embodiment, the cycloalkyl group is a saturated cyclic aliphatic hydrocarbon containing one or two rings, preferably one ring, and having 3, 4, 5, 6, 7, 8, 9, 10, 11 or 12 carbon atoms. In another embodiment, the cycloalkyl group is chosen from a C₃₋₈ cycloalkyl group. In another embodiment, the cycloalkyl group is chosen from a C₃₋₆ cycloalkyl group. Non-limiting exemplary cycloalkyl groups include cyclopropyl, cyclobutyl, cyclopentyl, cyclohexyl, cycloheptyl, cyclooctyl, norbornyl, decalin, adamantyl, cyclohexenyl, and the like.

For the purpose of the present disclosure, the term "(cycloalkyl)alkyl" as used by itself or as part of another group refers to an alkyl group substituted with at least one optionally substituted cycloalkyl group. Non-limiting exemplary (cycloalkyl)alkyl groups include:

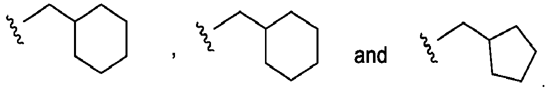

For the purpose of the present disclosure, the term "hydroxy(cycloalkyl)alkyl" as used by itself or as part of another group refers to (cycloalkyl)alkyl group substituted with at least one hydroxy group. The hydroxy group(s) can be at any available position. Non-limiting exemplary hydroxy(cycloalkyl)alkyl groups include:

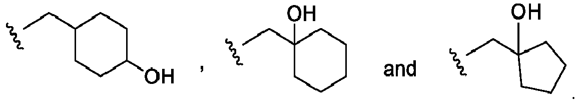

For the purpose of the present disclosure, the term "alkoxy" as used by itself or as part of another group refers to an optionally substituted alkyl, optionally substituted cycloalkyl, optionally substituted alkenyl or optionally substituted alkynyl attached to a terminal oxygen atom. In one embodiment, the alkoxy group is chosen from a C₁₋₄ alkoxy group. In another embodiment, the alkoxy group is chosen from a C₁₋₄ alkyl attached to a terminal oxygen atom, *e.g*., methoxy, ethoxy, and *tert*-butoxy.

For the purpose of the present disclosure, the term "alkylthio" as used by itself or as part of another group refers to a sulfur atom substituted by an optionally substituted alkyl group. In one embodiment, the alkylthio group is chosen from a C₁₋₄ alkylthio group. Non-limiting exemplary alkylthio groups include -SCH₃, and -SCH₂CH₃.

For the purpose of the present disclosure, the term "alkoxyalkyl" as used by itself or as part of another group refers to an alkyl group substituted with an alkoxy group. Non-limiting exemplary alkoxyalkyl groups include methoxymethyl, methoxyethyl, methoxypropyl, methoxybutyl, ethoxymethyl, ethoxyethyl, ethoxypropyl, ethoxybutyl, propoxymethyl, iso-propoxymethyl, propoxyethyl, propoxypropyl, butoxymethyl, tert-butoxymethyl, isobutoxymethyl, sec-butoxymethyl, and pentyloxymethyl.

For the purpose of the present disclosure, the term "haloalkoxy" as used by itself or as part of another group refers to a haloalkyl attached to a terminal oxygen atom. Non-limiting exemplary haloalkoxy groups include fluoromethoxy, difluoromethoxy, trifluoromethoxy, and 2,2,2-trifluoroethoxy.

"(5 to 6 membered)heteroaryl" either refers to groups containing an aromatic ring containing 5 to 6 ringatoms, wherein one or two or three, preferably one, of said ringatoms is a heteroatom selected from nitrogen, oxygen and sulphur; the remainder of the ringatoms being carbon atoms. Preferred (5 to 6
membered)heteroaryl are thiophene, benzothiophene, benzofuran, benzimidazole, benzoxazole, benzothiazole, benzisothiazole, naphtho[2,3-b]thiophene, furan, isoindolizine, xantholene, phenoxatine, pyrrole, imidazole, pyrazole, pyridine, pyrazine, pyrimidine, pyridazine, indole, isoindole, 1H-indazole, purine, quinoline, isoquinoline, phthalazine, naphthyridine, quinoxaline, cinnoline, carbazole, phenanthridine, acridine, phenazine, thiazole, isothiazole, phenothiazine, oxazole, isooxazole,furazane, phenoxazine, 2-,3- or4 -pyridyl, 2-, 3-, 4-, 5-, or 8- quinolyl, 1-, 3-, 4-, or 5- isoquinolinyl1-, 2-, or 3- indolyl, and 2-, or 3-thienyl. The group may be a terminal group or a bridging group.

For the purpose of the present disclosure, the term "(C₁-C₁₀)alkyl" as used by itself or as part of another group refers to a straight- or branched-chain aliphatic hydrocarbon containing one to ten, i.e. 1, 2, 3, 4, 5, 6, 7, 8, 9, or 10, carbon atoms *(i.e.,* C₁-C₁₀ alkyl) or the number of carbon atoms designated (*i.e.,* a C₁ alkyl such as methyl, a C₂ alkyl such as ethyl, a C₃ alkyl such as propyl or isopropyl, etc.). In one embodiment, the alkyl group is chosen from a straight chain (C₁-C₁₀)alkyl group. In another embodiment, the alkyl group is chosen from a branched chain (C₃-C₁₀)alkyl group. In another embodiment, the alkyl group is chosen from a straight chain (C₁-C₆)alkyl group. In another embodiment, the alkyl group is chosen from a branched chain (C₃-C₆)alkyl group. In another embodiment, the alkyl group is chosen from a straight chain (C₁-C₄)alkyl group. In another embodiment, the alkyl group is chosen from a branched chain (C₃-C₄)alkyl group. In another embodiment, the alkyl group is chosen from a straight or branched chain (C₃-C₄)alkyl group. Non-limiting exemplary (C₁-C₁₀)alkyl groups include methyl, ethyl, propyl, isopropyl, butyl, *sec-*butyl, *tert*-butyl, *iso*-butyl, 3-pentyl, hexyl, heptyl, octyl, nonyl, decyl, and the like. Non-limiting exemplary C₁₋₄ alkyl groups include methyl, ethyl, propyl, isopropyl, butyl, *sec*-butyl, *tert*-butyl, and *iso*-butyl.

The term "linear or branched (C₁-C₆)alkyl" as used herein refers to an aliphatic hydrocarbon group which may be straight or branched having 1 to 6 carbon atoms in the chain, preferably 1 to 4 carbons. Preferred linear or branched (C₁-C₆)alkyl is methyl, ethyl, n-propyl, i-propyl, n-butyl, i-butyl or t-butyl. The letter is also referred to as tBu. The group may be a terminal group or a bridging group.

As used herein, the term "absent" means that a group is not present in a compound. Instead, the absent group is a single bond.

"Heteroalkyl" refers to a straight- or branched-chain alkyl group preferably having from 2 to 14 carbons, more preferably 2 to 10 carbons in the chain, one or more of which has been replaced by a heteroatom selected from S, O, and N. Exemplary heteroalkyls include alkyl ethers, secondary and tertiary alkyl amines, amides, alkyl sulfides, and the like. The group may be a terminal group or a bridging group. As used herein reference to the normal chain when used in the context of a bridging group refers to the direct chain of atoms linking the two terminal positions of the bridging group. Non-limiting exemplary heteroalkyl groups include: -CH₂N(H)CH₂CH₂N(CH₃)₂; -CH₂N(CH₃)CH₂CH₂N(CH₃)₂; -CH₂N(H)CH₂CH₂C H₂N(CH₃)₂; -CH₂N(H)CH₂CH₂OH; -CH₂N(CH₃)CH₂CH₂OH; -CH₂OCH₂CH₂OCH₃, -O CH₂CH₂OCH₂CH₂OCH₃; -CH₂NHCH₂CH₂OCH₂; -OCH₂CH₂NH₂; and -NHCH₂CH₂N(H)CH₃.

As used herein, the term "unsubstituted" means that there is no substituent or that the only substituents are hydrogen.

The term "substituted" as used throughout the specification denotes that the group contains one or more substituent groups. Preferably the substituent groups are one or more groups independently selected from the group consisting of halogen, =O, =S, -CN, -NO₂, -CF₃, -OCF₃, alkyl, alkenyl, alkynyl, haloalkyl, haloalkenyl, haloalkynyl, heteroalkyl, cycloalkyl, cycloalkenyl, heterocycloalkyl, heterocycloalkenyl, aryl, heteroaryl, cycloalkylalkyl, heterocycloalkylalkyl, heteroarylalkyl, arylalkyl, cycloalkylalkenyl, heterocycloalkylalkenyl, arylalkenyl, heteroarylalkenyl, cycloalkylheteroalkyl, heterocycloalkylheteroalkyl, arylheteroalkyl, heteroarylheteroalkyl, hydroxy, hydroxyalkyl; alkoxy, alkoxyalkyl, alkoxycycloalkyl, alkoxyheterocycloalkyl, alkoxyaryl, alkoxyheteroaryl, alkoxycarbonyl, alkylaminocarbonyl, alkenyloxy, alkynyloxy, cycloalkyloxy, cycloalkenyloxy, heterocycloalkyloxy, heterocycloalkenyloxy, aryloxy, phenoxy, benzyloxy, heteroaryloxy,arylalkyloxy, arylalkyl, heteroarylalkyl, cycloalkylalkyl, heterocycloalkylalkyl, arylalkyloxy, amino, alkylamino, acylamino, aminoalkyl, arylamino, sulfonylamino, sulfinylamino, sulfonyl, alkylsulfonyl, arylsulfonyl, aminosulfonyl, sulfinyl, alkylsulfinyl, arylsulfinyl, aminosulfinylaminoalkyl, and -COOH.

For the purpose of the present disclosure, the term "optionally substituted alkyl" as used by itself or as part of another group means that the alkyl as defined above is either unsubstituted or substituted with one, two, or three substituents independently chosen from nitro, haloalkoxy, aryloxy, aralkyloxy, alkylthio, sulfonamido, alkylcarbonyl, arylcarbonyl, alkylsulfonyl, arylsulfonyl, ureido, guanidino, carboxy, carboxyalkyl, cycloalkyl, and the like. In one embodiment, the optionally substituted alkyl is substituted with two substituents. In another embodiment, the optionally substituted alkyl is substituted with one substituent. Non-limiting exemplary optionally substituted alkyl groups include -CH₂CH₂NO₂, - CH₂CH₂CO₂H, -CH₂CH₂SO₂CH₃, -CH₂CH₂COPh, -CH₂C₆H₁₁, and the like.

The term "electron withdrawing group" as used herein refers to an atom or group that draws electron density from neighboring atom(s) towards itself, usually by resonance or inductive effects. Preferred examples of electron withdrawing groups are -CI, -Br, -O-C₆F₅H, -O(CO)-(C₁-C₆)alkyl, and -OH.

The term "ω-amino acid" has a general structure of 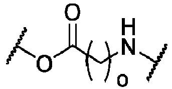 with o being an integer of 1, 2, 3, 4, 5, 6, or 7. Preferred examples of ω-amino acid are glycine, 2-aminoacetic acid, 3-aminopropionic acid, 4-aminobutyric acid, 4-aminovaleric acid, and 6-aminohexanoic acid.

As used herein, the term "dipeptide" relates to a peptide consisting of two canonical amino acids.

As used herein, the term "tripeptide" relates to a peptide consisting of three canonical amino acids.

As used herein, the term "divalent linker group" relates to a group having a chemical valence of two. In other words, a divalent linker group is bonded to two groups. According to one embodiment, the divalent linker group is bonded to group A and to group Q. A divalent linker group according to the present disclosure can be a linear or branched (C₁-C₁₀) alkyl, substituted or unsubstituted aryl, substituted or unsubstituted heteroaryl, (5 to 8 membered)heterocyclyl, (5 to 8 membered)heterocyclyl substituted with (C₁-C₁₀)alkyl. Preferred divalent linker groups are -CH₂-CH₂-, -CH₂-CH₂-CH₂-, -CH₂-CH₂-CH₂-CH₂-, -CH₂-CH₂-CH₂-CH₂-CH₂-, or -CH₂-CH₂-CH₂-CH₂-CH₂-CH₂-.

As used herein the term "PEG linker" is a group of the formula 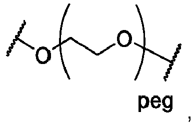 wherein peg is 1, 2, 3, 4, or 5; preferably peg is 2 or 3.

The terms "a" and "an" refer to one or more.

### Examples

### Chemistry

All starting materials as used herein were obtained from commercial suppliers.

### Synthesis of Compound of formula (¹⁹F-Va)

The synthesis of compound of formula (Va) was carried out as follows:

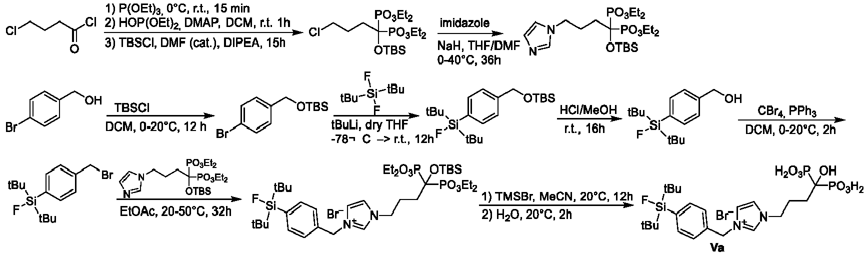

### tetraethyl (1-((tert-butyldimethylsilyl)oxy)-4-chlorobutane-1,1-diyl)bis (phosphonate)

To a stirring 4-chlorobutanoyl chloride (50 g, 354.62 mmol) was added dropwise triethyl phosphite (58.92 g, 354.62 mmol) at 0 °C under N₂, then the mixture was allowed to warm to 20 °C and stirred for 15 min. The mixture was diluted with DCM (1000 mL) and then 1-ethoxyphosphonoyloxyethane (48.97 g, 354.62 mmol) was added at 0 °C. The mixture was stirred at 20 °C for 15 min and then added with DMAP (43.32 g, 354.62 mmol) at 20 °C and stirred for 1 h. The mixture was cooled to 0 °C and added with TBSCI (58.79 g, 390.08 mmol), warmed to 20 °C and stirred for 15 h. The reaction mixture was washed by H₂O (300 mL), sat. -NH₄Cl (300 mL), dried over Na₂SO₄, filtered and concentrated under reduced pressure. The residue was purified by prep-HPLC with the following conditions: column: Phenomenex luna c18 250mm*100mm*15um; mobile phase: [water(FA)-ACN]; B%: 60%-90%, 20 min to give a mixture of tetraethyl (1-((*tert*butyldimethylsilyl)oxy)-4-chlorobutane-1,1-diyl)bis(phosphonate) and (*Z*)-diethyl (1-((*tert*-butyldimethylsilyl)oxy)-4-chlorobut-1-en-1-yl)phosphonate (71 g) as yellow oil. ¹H NMR (400 MHz, Chloroform-d) δ 4.20 (m, 8H), 3.58-3.48 (m, 2H), 2.26-2.10 (m, 4H), 1.33 (t, *J* = 7.2 Hz, 12H), 0.92 (d, *J* = 24.0 Hz, 9H), 0.19 (s, 6H). LC-MS calculated for C18H41ClO7P2Si m/z 494.18 [M+H]⁺, found 495.2.

### tetraethyl (1-((tert-butyldimethylsilyl)oxy)-4-(1H-imidazol-1-yl)butane-1,1-diyl)bis(phosphonate)

To a solution of NaH (5.66 g, 141.41 mmol, 60% purity) in THF (200 mL) and DMF (200 mL) was added imidazole (9.63 g, 141.41 mmol) at 0 °C. The mixture was stirred at 20 °C for 1 h. Tetraethyl (1-((*tert*-butyldimethylsilyl)oxy)-4-chlorobutane-1,1-diyl)bis(phosphonate) (70 g, 141.41 mmol) and Nal (10.60 g, 70.71 mmol) were added at 0 °C and the mixture was stirred at 40 °C for 36 h. The mixture was quenched by sat. NH₄Cl (50 mL) and extracted with EtOAc (3 × 50 mL). The combined organic layers were washed with brine (50 mL), dried over Na₂SO₄, filtered and concentrated under reduced pressure. The crude product was purified by reversed-phase MPLC (neutral) to give tetraethyl (1-((*tert*-butyldimethylsilyl)oxy)-4-(1*H*-imidazol-1-yl)butane-1,1-diyl)bis(phosphonate) (6 g, 8%) as yellow oil. ¹H-NMR (400 MHz, CDCl₃) δ 7.56 (s, 1H), 7.08 (s, 1H), 6.95-6.92 (m, 1H), 4.25-4.12 (m, 8H), 3.96 (t, *J* = 6.8 Hz, 2H), 2.14 (br s, 4H), 1.32 (dt, *J* = 2.4, 7.2 Hz, 12H), 0.88 (s, 9H), 0.14 (s, 6H). LC-MS calculated for C₂₁H₄₄N₂O₇P₂Si m/z 526.27 [M+H]⁺, found 527.1.

### ((4-bromobenzyl)oxy)(tert-butyl)dimethylsilane

To a solution of (4-bromophenyl)methanol (50 g, 267.33 mmol) in DCM (600 mL) was added imidazole (27.30 g, 401.00 mmol) and TBSCI (60.44 g, 401.00 mmol) at 0 °C. The mixture was stirred at 20 °C for 12 h. The mixture was diluted with H₂O (400 mL), and extracted with DCM (3 × 100 mL). The combined organic layers were washed with brine (300 mL), dried over Na₂SO₄, filtered and concentrated under reduced pressure. The residue was purified by silica gel column chromatography (Petroleum ether: EtOAc = 1:0 to 10:1) to give ((4-bromobenzyl)oxy)(*tert*-butyl)dimethylsilane (77 g, 255.56 mmol, 96 %) as colorless oil. ¹H-NMR (400 MHz, CDCl₃) δ 7.46 (d, *J* = 8.4 Hz, 2H), 7.21 (d, *J* = 8.4 Hz, 2H), 4.69 (s, 2H), 0.95 (s, 9H), 0.11 (s, 6H).

### di-tert-butyl(4-(((tert-butyldimethylsilyl)oxy)methyl)phenyl)fluorosilane

To a solution of ((4-bromobenzyl)oxy)(*tert-*butyl)dimethylsilane (30 g, 99.57 mmol) in THF (300 mL) was added *t*-BuLi (1.3 M, 176.16 mL, 229.00 mmol) at -78 °C. The mixture was stirred at -78 °C for 0.5 h and then added dropwise to a solution of di-*tert*-butyldifluorosilane (21.54 g, 119.48 mmol) in THF (200 mL) at -78 °C. The mixture was stirred at 20 °C for 12 h. The reaction mixture was quenched by pouring into sat. NH₄Cl (500 mL) at 0 °C, and extracted with MTBE (3 × 200 mL). The combined organic layers were washed with brine (300 mL), dried over Na₂SO₄, filtered and concentrated under reduced pressure to give di-*tert*-butyl(4-(((*tert*butyldimethylsilyl)oxy)methyl)phenyl)fluorosilane (43 g, crude) as colorless oil. ¹H-NMR (400 MHz, CDCl₃) δ 7.52 (d, *J* = 8.0 Hz, 2H), 7.29 (d, *J* = 6.4 Hz, 2H), 4.71 (s, 2H), 1.00 (s, 18H), 0.90 (s, 9H), 0.06 (s, 6H).

### (4-(di-tert-butylfluorosilyl)phenyl)methanol

To a solution of di-*tert*-butyl(4-(((*tert*-butyldimethylsilyl)oxy)methyl)phenyl) fluorosilane (43 g, 112.36 mmol) in MeOH (300 mL) was added HCI (12 M, 3 mL) at 20 °C. The mixture was stirred at 20 °C for 12 h. The reaction mixture was concentrated under reduced pressure. The residue was purified by silica gel column (Petroleum ether: EtOAc = 1:0 to 3:1) to give (4-(di-*tert*-butylfluorosilyl)phenyl) methanol (14.8 g, 49% yield) as white solid. ¹H-NMR (400 MHz, CDCl₃) δ 7.62 (d, *J* = 8.0 Hz, 2H), 7.40 (d, *J* = 8.0 Hz, 2H), 4.73 (s, 2H), 1.07 (d, *J* = 0.8 Hz, 18H).

### (4-(bromomethyl)phenyl)di-tert-butylfluorosilane

To a mixture (4-(di-*tert*-butylfluorosilyl)phenyl)methanol (14.8 g, 55.13 mmol) and CBr₄ (21.94 g, 66.16 mmol) in DCM (200 mL) was added a solution of PPh₃ (17.35 g, 66.16 mmol) in DCM (50 mL) at 0 °C under N₂. The reaction mixture was stirred at 20 °C for 2 h. The reaction mixture was concentrated under reduced pressure. The residue was purified by silica gel column (Petroleum ether:EtOAc = 1:0 to 10:1) to give (4-(bromomethyl)phenyl)di-*tert*-butylfluorosilane (21 g, 95.8% yield) as white solid. ¹H-NMR (400 MHz, CDCl₃) δ 7.59 (d, *J* = 8.0 Hz, 2H), 7.41 (d, *J* = 8.0 Hz, 2H), 4.51 (s, 2H), 1.06 (d, *J* = 1.2 Hz, 18H).

### 1-(4-((tert-butyldimethylsilyl)oxy)-4,4-bis(diethoxyphosphoryl)butyl)-3-(4-(di-tert-butylfluorosilyl)benzyl)-1H-imidazol-3-ium hydrobromate

To a solution of (4-(bromomethyl)phenyl)di-*tert*-butylfluorosilane (200 mg, 0.60 mmol) in EtOAc (5 mL) was added tetraethyl (1-((*tert*-butyldimethylsilyl)oxy)-4-(1*H*-imidazol-1-yl)butane-1,1-diyl)bis(phosphonate) (477 mg, 0.90 mmol) at 20 °C. The mixture was stirred at 50 °C for 32 h. The mixture was filtered. And the filter cake was dried under reduced pressure to tive 1-(4-((*tert-*butyldimethylsilyl)oxy)-4,4-bis(diethoxyphosphoryl)butyl)-3-(4-(di-*tert*-butylfluorosilyl)benzyl)-1*H*-imidazol-3-ium hydrobromate (700 mg, crude) as white solid. LC-MS calculated for C₃₆H₆₈FN₂O₇P₂Si₂⁺ m/z 777.40 [M]⁺, found 777.4.

### 3-(4-(di-tert-butylfluorosilyl)benzyl)-1-(4-hydroxy-4,4-diphosphonobutyl)-1H-imidazol-3-ium bromide (Va)

To a solution of 1-(4-((*tert*-butyldimethylsilyl)oxy)-4,4-bis(diethoxyphosphoryl) butyl)-3-(4-(di-*tert*-butylfluorosilyl)benzyl)-1*H*-imidazol-3-ium hydrobromate (300 mg, 0.349 mmol) in ACN (6 mL) was added TMSBr (3.54 g, 0.023 mmol) at 20 °C. The mixture was stirred at 20 °C for 12 h. H₂O (2 mL) was added and the mixture was stirred at 20°C for 2 h. The mixture was concentrated under reduced pressure. The residue was purified by prep-HPLC with the following conditions: column: Phenomenex luna C18 100*40mm*3 µm; mobile phase: [water(TFA)-ACN]; B%: 25%-55%, 8 min to give 3-(4-(di-*tert*-butylfluorosilyl)benzyl)-1-(4-hydroxy-4,4-diphosphonobutyl)-1*H*-imidazol-3-ium bromide (48.35 mg) as white solid. ¹H-NMR (400 MHz, DMSO) δ 9.38 (br s, 1H), 7.81 (br d, *J* = 12.4 Hz, 2H), 7.62 (br d, *J* = 4.4 Hz, 2H), 7.41 (br d, *J* = 4.8 Hz, 2H), 5.48 (br s, 2H), 4.22 (br s, 2H), 2.24-2.03 (m, 2H), 1.97-1.73 (m, 2H), 1.26-0.76 (m, 18H) LC-MS calculated for C₂₂H₃₈FN₂O₇P₂Si⁺ m/z 551.19 [M]+, found 551.1.

### Radiolabeling protocol of (Va)

The synthesis scheme for preparation of (Va) was performed as follows:

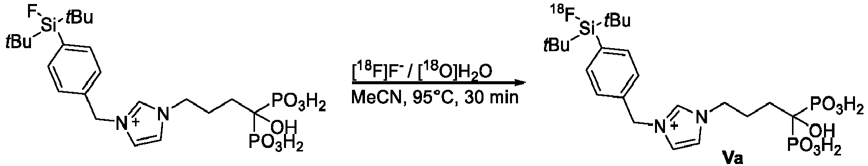

### Method 1

[¹⁸F]HF in [¹⁸O]H₂O obtained from cyclotron (200-500 µL) was added to a solution of FSi-Zole precursor in DMSO (50-100 µL). The resulting solution was heated at 95°C for 30 min. The reaction solution was diluted with 1.5 mL 70% acetonitrile/water and purified via HPLC: column COSMOSIL 5C18-ARII 6.0ID*150 mm; mobile phase: A: H₂O + 0.1%TFA, B: acetonitrile + 0.1% TFA; method: 0->20 min, 5%->90% B, 20-25 min 90% B, 25-27 min, 90%->5% B, 27-30 min, 5% B, flow rate 1.5 mL/min, retention time 15.6 ± 0.5 min. The collected fraction was diluted with PBS until 2.5 mL and applied to a preconditioned PD-10 desalting column, elute the column with PBS buffer with 0.5 mL each fraction. The fractions containing radioactivity were combined and passed through a 0.2 µM sterile filter, and was ready for ex vivo and in vivo application.

### Method 2

[¹⁸F]HF in [¹⁸O]H₂O obtained from cyclotron was passed through an anion exchange cartridge (Waters QMA cartridge preconditioned by 15 mL pure water). The cartridge was rinsed with 2 mL pure water and was dried with 10 mL air. A solution containing potassium carbonate and Kryptofix 222 (300 µL, 0.025 M/0.05 M, respectively, in acetonitrile/water 3:1) was used to elute the [¹⁸F]F⁻ from the cartridge. The solution was dried azeotropically under nitrogen flow at 120°C. The procedure was repeated twice after the addition of anhydrous acetonitrile (0.5 mL). A solution of anhydrous acetonitrile (200 µL) and FSi-Zole precursor (50 µL, 1 mg/mL solution in dry DMSO) was added to the residue after concentration, followed by reaction at room temperature for 15 min. The reaction solution was diluted with 1.5 mL 70% acetonitrile/water and purified via HPLC: column COSMOSIL 5C18-ARII 6.0ID*150 mm; mobile phase: A: H₂O + 0.1%TFA, B: acetonitrile + 0.1% TFA; method: 0->20 min, 5%->90% B, 20-25 min 90% B, 25-27 min, 90%->5% B, 27-30 min, 5% B, flow rate 1.5 mL/min, retention time 15.6 ± 0.5 min. The collected fraction was diluted with PBS until 2.5 mL and applied to a preconditioned PD-10 column, elute the column with PBS buffer with 0.5 mL each fraction. The fractions containing radioactivity were combined and passed through a 0.2 µM sterile filter, and was ready for ex vivo and in vivo application.

### Method 3

[¹⁸F]HF in [¹⁸O]H₂O obtained from cyclotron was passed through an anion exchange cartridge (Waters QMA cartridge preconditioned by 15 mL pure water). The cartridge was rinsed with 2 mL pure water and was dried with 10 mL air. A solution of Et₄NHCO₃ aq. (0.075M, 80µL) together with water (120 µL) and acetonitrile (400 µL) was used to elute the [¹⁸F]F⁻ from the cartridge. The solution was dried azeotropically under nitrogen flow at 120°C. The procedure was repeated twice after the addition of anhydrous acetonitrile (0.5 mL). A solution of anhydrous acetonitrile (200 µL) and FSi-Zole precursor (50 µL, 1 mg/mL solution in dry DMSO) was added to the residue after concentration, followed by reaction at room temperature for 15 min. The reaction solution was diluted with 1.5 mL water and trapped on a Waters Sep-Pak tC18 cartridge (preconditioned by 5 mL ethanol and 5 mL water). The cartridge was washed with 5 mL water, dried with 10 mL air and eluted with ethanol/PBS (2 mL, 1:1 mixture). Ethanol was removed by nitrogen flow and the residue was diluted with PBS buffer. The solution was passed through a 0.2 µM sterile filter, which was then ready for ex vivo and in vivo application.

### Synthesis of Compound of formula (¹⁹F-IVa)

The synthesis of compound of formula (¹⁹F-IVa) was carried out as follows:

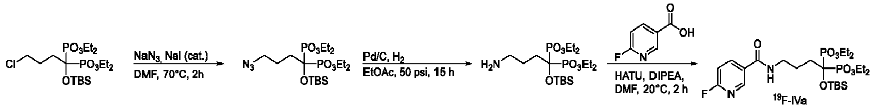

### tetraethyl (4-azido-1-((tert-butyldimethylsilyl)oxy)butane-1,1-diyl)bis(phosphonate)

A mixture of diethyl diethyl 1-[(tert-butyldimethylsilyl)oxy]-4-chloro-1-(diethoxyphosphoryl)butylphosphonate (13.6 g, 27.475 mmol, 1.00 equiv) and azido sodium (3.57 g, 54.950 mmol, 2 eq.) in anhydrous DMF (100.00 mL) was stirred for 4 hours at 80 °C under nitrogen atmosphere. The reaction was quenched with water (100 mL) at 25 °C. The resulting mixture was extracted with DCM (3 × 200 mL). The combined organic layers were washed with brine (5 × 100 mL), dried over anhydrous Na₂SO₄. After filtration, the filtrate was concentrated under reduced pressure to afford diethyl diethyl 4-azido-1-[(tert-butyldimethylsilyl)oxy]-1-(diethoxyphosphoryl)butylphosphonate (13 g, crude) as a yellow oil. The crude product was used for next step without further purification. LC-MS calculated for C₁₈H₄₁N₃O₇P₂Si m/z 502.57 [M+H]⁺, found 502.3.

### tetraethyl (4-amino-1-((tert-butyldimethylsilyl)oxy)butane-1,1-diyl)bis(phosphonate)

To a solution of diethyl 4-azido-1-[(tert-butyldimethylsilyl)oxy]-1-(diethoxyphosphoryl)butylphosphonate (13 g, 25.91 mmol, 1.00 eq.) in 200 mL ethyl acetate was added Pd/C (10%, 1.38 g) in a pressure tank. The mixture was hydrogenated at room temperature under 50 psi of hydrogen pressure for 15 hours, filtered through a Celite pad and concentrated under reduced pressure. The resulting mixture was concentrated under reduced pressure to afford diethyl 4-amino-1-[(tert-butyldimethylsilyl)oxy]-1-(diethoxyphosphoryl)butylphosphonate (10 g, crude) as a yellow oil. ¹H-NMR (400 MHz, DMSO) δ 4.06 (m, 8H), 2.53 (m, 2H), 2.04 (m, 2H), 1.64 (m, 2H), 1.25 (dt, 12H), 0.87 (s, 9H), 0.16 (s, 6H). LCMS calculated for C₁₈H₄₃NO₇P₂Si m/z 476.57 [M+H]⁺, found 476.3.

### tetraethyl (1-((tert-butyldimethylsilyl)oxy)-4-(6-fluoronicotinamido) butane-1,1-diyl)bis(phosphonate)

To a stirred solution of diethyl 4-amino-1-[(tert-butyldimethylsilyl)oxy]-1-(diethoxyphosphoryl)butylphosphonate (1.69 g, 3.544 mmol, 1 equiv) and 6-fluoropyridine-3-carboxylic acid (500 mg, 3.544 mmol, 1.00 equiv) in DMF (10 mL) were added HATU (2.02 g, 5.316 mmol, 1.50 equiv) and DIEA (1.25 mL, 7.194 mmol, 2.03 equiv) at 20 degrees C. The reaction was stirred for 2 hours at 20 degrees C. The reaction was quenched with water. The aqueous layer was extracted with EA (3x100 mL).The resulting mixture was concentrated under reduced pressure. The residue was purified by silica gel column chromatography, eluted with MeOH in EA (7%) to afford diethyl 1-[(tert-butyldimethylsilyl)oxy]-1-(diethoxyphosphoryl)-4-[(6-fluoropyridin-3-yl)formamido]butylphosphonate (691 mg, 32.57%) as a yellow oil. ¹H NMR (400 MHz, Methanol-*d*₄) δ 8.64 (d, *J* = 2.4 Hz, 1H), 8.37 - 8.28 (m, 1H), 7.17 (dd, *J* = 8.8, 2.4 Hz, 1H), 4.30 - 4.10 (m, 9H), 3.40 (t, *J* = 6.4 Hz, 2H), 2.25 - 2.05 (m, 2H), 2.05 - 1.92 (m, 2H), 1.36 - 1.30 (m, 12H), 0.91 (s, 9H), 0.21 (s, 6H). LCMS calculated for C₂₄H₄₅FN₂O₈P₂Si m/z 599.24 [M+H]⁺, found 599.3.

### Radiolabeling protocol of (IVa)

Radiolabeling was carried out as follows:

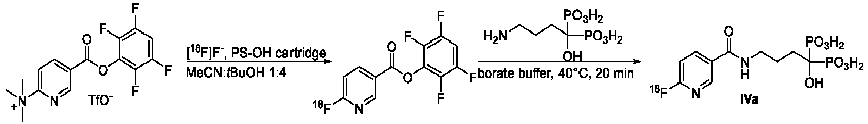

### Method 1

[¹⁸F]HF in [¹⁸O]H₂O obtained from cyclotron was passed through an anion-exchange cartridge (Chromafix PS-OH, prewetted with 10 mL distilled water). The original vial was rinsed with 2 mL pure water and also applied to the cartridge. The cartridge was washed with 3 mL anhydrous acetonitrile and dried with nitrogen flow for 5 min. The [¹⁸F]F⁻ was slowly eluted with a solution of quaternary ammonium triflate precursor (5-10 mg) in anhydrous isobutanol:acetonitrile (4:1, 1 mL) for over 5 min. The eluate was diluted with 8 mL water and trapped on an AFFINIMIP^{®} SPE ¹⁸F-aromatic nucleophilic substitution cartridge (preconditioned by 2 mL acetonitrile). The cartridge was washed with 5 mL of water and elute with 1.5 mL of 65% acetonitrile/water. An aliquot of 0.5-1 MBq of the eluate was removed for quality control using HPLC: column COSMOSIL 5C18-ARII 6.0ID*150 mm; mobile phase: A: H₂O + 0.1% TFA, B: acetonitrile + 0.1% TFA; method: 0->20 min, 30%->80% B, 20-22 min 80%->95% B, 22-27 min, 95% B, 27-30 min, 95%->30% B, flow rate 2.0 mL/min, retention time 15.2 min. To the eluate was added a solution of alendronate in borate buffer (pH 9.5, 200 µL) and the resulting solution was heated at 40°C for 20 min. The reaction solution was diluted with 1 mL PBS buffer and purified via HPLC: column COSMOSIL 5C18-PAQ 101D*250 mm; mobile phase: A: PBS, B: ethanol; method: 0->5 min, 5% B, 5-20 min 5%->35% B, 20-23 min, 35%->90% B, 23-28 min, 90% B, 28-33 min, 90%->5% B, 33-40min, 5% B, flow rate 3.0 mL/min, retention time 6.0 ± 0.5 min. The collected fraction was filtered through a 0.2 µM sterile filter and was ready for ex vivo and in vivo application.

### Method 2

[¹⁸F]HF in [¹⁸O]H₂O obtained from cyclotron was passed through an anion-exchange cartridge (Chromafix PS-OH, prewetted with 10 mL distilled water). The original vial was rinsed with 2 mL pure water and also applied to the cartridge. The cartridge was washed with 3 mL anhydrous acetonitrile and dried with nitrogen flow for 5 min. The [¹⁸F]F⁻ was slowly eluted with a solution of quaternary ammonium triflate precursor (5-10 mg) in anhydrous isobutanol:acetonitrile (4:1, 1 mL) for over 5 min. The eluate was diluted with 8 mL water and trapped on an AFFINIMIP^{®} SPE ¹⁸F-aromatic nucleophilic substitution cartridge (preconditioned by 2 mL acetonitrile). The cartridge was washed with 5 mL of water and elute with 1.0 mL of acetone. An aliquot of 0.5-1 MBq of the eluate was removed for quality control using HPLC: column COSMOSIL 5C18-ARII 6.0ID*150 mm; mobile phase: A: H₂O + 0.1% TFA, B: acetonitrile + 0.1% TFA; method: 0->20 min, 30%->80% B, 20-22 min 80%->95% B, 22-27 min, 95% B, 27-30 min, 95%->30% B, flow rate 2.0 mL/min, retention time 15.2 min. The solvent was removed under nitrogen flow at room temperature. To the residue after concentration was added a solution of alendronate in borate buffer (pH 9.5, 200 µL) and the resulting solution was heated at 40°C for 20 min. The reaction solution was diluted with 1 mL PBS buffer and purified via HPLC: column COSMOSIL 5C18-PAQ 101D*250 mm; mobile phase: A: PBS, B: ethanol; method: 0->5 min, 5% B, 5-20 min 5%->35% B, 20-23 min, 35%->90% B, 23-28 min, 90% B, 28-33 min, 90%->5% B, 33-40min, 5% B, flow rate 3.0 mL/min, retention time 6.0 ± 0.5 min. The collected fraction was filtered through a 0.2 µM sterile filter and was ready for ex vivo and in vivo application.

### Method 3

[¹⁸F]HF in [¹⁸O]H₂O obtained from cyclotron was passed through an anion-exchange cartridge (Chromafix PS-OH, preconditioned with 5 mL 0.1M NaHCO₃ and 5 mL water). The original vial was rinsed with 2 mL pure water and also applied to the cartridge. The cartridge was washed with 3 mL anhydrous acetonitrile and dried with nitrogen flow for 5 min. The [¹⁸F]F⁻ was slowly eluted with a solution of quaternary ammonium triflate precursor (5-10 mg) in anhydrous isobutanol:acetonitrile (4:1, 1 mL) for over 5 min. The eluate was diluted with 8 mL water and trapped on a Waters Oasis MCX cartridge (preconditioned by 5 mL ethanol and 5 mL water). The cartridge was washed with 5 mL of water and elute with 1.5 mL 65% acetonitrile/water. An aliquot of 0.5-1 MBq of the eluate was removed for quality control using HPLC: column COSMOSIL 5C18-ARII 6.0ID*150 mm; mobile phase: A: H₂O + 0.1% TFA, B: acetonitrile + 0.1% TFA; method: 0->20 min, 30%->80% B, 20-22 min 80%->95% B, 22-27 min, 95% B, 27-30 min, 95%->30% B, flow rate 2.0 mL/min, retention time 15.2 min. To the eluate was added a solution of alendronate in borate buffer (pH 9.5, 200 µL) and the resulting solution was heated at 40°C for 20 min. The reaction solution was diluted with 1 mL PBS buffer and purified via HPLC: column COSMOSIL 5C18-PAQ 10ID*250 mm; mobile phase: A: saline, B: ethanol; method: 0->5 min, 5% B, 5-20 min 5%->35% B, 20-23 min, 35%->90% B, 23-28 min, 90% B, 28-33 min, 90%->5% B, 33-40min, 5% B, flow rate 3.0 mL/min, retention time 6.0 ± 0.5 min. The collected fraction was filtered through a 0.2 µM sterile filter and was ready for ex vivo and in vivo application.

### Method 4

[¹⁸F]HF in [¹⁸O]H₂O obtained from cyclotron was passed through an anion-exchange cartridge (Chromafix PS-OH, preconditioned with 5 mL 0.1M NaHCO₃ and 5 mL water). The original vial was rinsed with 2 mL pure water and also applied to the cartridge. The cartridge was washed with 3 mL anhydrous acetonitrile and dried with nitrogen flow for 5 min. The [¹⁸F]F⁻ was slowly eluted with a solution of quaternary ammonium triflate precursor (5-10 mg) in anhydrous isobutanol:acetonitrile (4:1, 1 mL) for over 5 min. The eluate was diluted with 8 mL water and trapped on a Waters Oasis MCX cartridge (preconditioned by 5 mL ethanol and 5 mL water). The cartridge was washed with 5 mL of water and elute with 1.0 mL acetonitrile. An aliquot of 0.5-1 MBq of the eluate was removed for quality control using HPLC: column COSMOSIL 5C18-ARII 6.0ID*150 mm; mobile phase: A: H₂O + 0.1% TFA, B: acetonitrile + 0.1% TFA; method: 0->20 min, 30%->80% B, 20-22 min 80%->95% B, 22-27 min, 95% B, 27-30 min, 95%->30% B, flow rate 2.0 mL/min, retention time 15.2 min. The solvent was removed by nitrogen flow at room temperature. A solution of alendronate in borate buffer (pH 9.5, 100 µL) was added to the residue, and the resulting solution was heated at 40°C for 20 min. The reaction solution was diluted with 1 mL PBS buffer and purified via HPLC: column COSMOSIL 5C18-PAQ 101D*250 mm; mobile phase: A: saline, B: ethanol; method: 0->5 min, 5% B, 5-20 min 5%->35% B, 20-23 min, 35%->90% B, 23-28 min, 90% B, 28-33 min, 90%->5% B, 33-40min, 5% B, flow rate 3.0 mL/min, retention time 6.0 ± 0.5 min. The collected fraction was filtered through a 0.2 µM sterile filter and was ready for ex vivo and in vivo application.

### Method 5

[¹⁸F]HF in [¹⁸O]H₂O obtained from cyclotron was passed through an anion-exchange cartridge (Chromafix PS-OH, prewetted with 10 mL distilled water). The original vial was rinsed with 2 mL pure water and also applied to the cartridge. The cartridge was washed with 3 mL anhydrous acetonitrile and dried with nitrogen flow for 5 min. The [¹⁸F]F⁻ was slowly eluted with a solution of quaternary ammonium triflate precursor (5-10 mg) in anhydrous isobutanol:acetonitrile (4:1, 1 mL) for over 5 min. The eluate was diluted with 8 mL water and passed through stacked Waters Sep-Pak Plus CM cartridge and PS-2 cartridge (each preconditioned by 5 mL ethanol and 5 mL water), subsequently. The cartridges were washed with 5 mL of water. PS-2 cartridge was eluted with 1.0 mL dichloromethane. An aliquot of 0.5-1

MBq of the eluate was removed for quality control using HPLC: column COSMOSIL 5C18-ARII 6.0ID*150 mm; mobile phase: A: H₂O + 0.1% TFA, B: acetonitrile + 0.1% TFA; method: 0->20 min, 30%->80% B, 20-22 min 80%->95% B, 22-27 min, 95% B, 27-30 min, 95%->30% B, flow rate 2.0 mL/min, retention time 15.2 min. The solvent was removed by nitrogen flow at room temperature. A solution of alendronate in borate buffer (pH 9.5, 100 µL) was added to the residue, and the resulting solution was heated at 40°C for 20 min. The reaction solution was diluted with 1 mL PBS buffer and purified via HPLC: column COSMOSIL 5C18-PAQ 10ID*250 mm; mobile phase: A: PBS, B: ethanol; method: 0->5 min, 5% B, 5-20 min 5%->35% B, 20-23 min, 35%->90% B, 23-28 min, 90% B, 28-33 min, 90%->5% B, 33-40min, 5% B, flow rate 3.0 mL/min, retention time 6.0 ± 0.5 min. The collected fraction was filtered through a 0.2 µM sterile filter and was ready for ex vivo and in vivo application.

### Radiolabeling protocol of (IVb)

The synthesis scheme for preparation of (IVb) as follows:

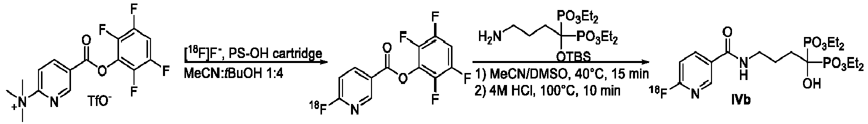

[¹⁸F]HF in [¹⁸O]H₂O obtained from cyclotron was passed through an anion-exchange cartridge (Chromafix PS-OH, prewetted with 10 mL distilled water). The original vial was rinsed with 2 mL pure water and also applied to the cartridge. The cartridge was washed with 3 mL anhydrous acetonitrile and dried with nitrogen flow for 5 min. The [¹⁸F]F⁻ was slowly eluted with a solution of quaternary ammonium triflate precursor (5-10 mg) in anhydrous isobutanol:acetonitrile (4:1, 1 mL) for over 5 min. The eluate was diluted with 8 mL water and trapped on stacked Waters Sep-Pak Plus CM and PS-2 cartridges (each preconditioned by 5 mL ethanol and 5 mL water). The cartridges were washed with 5 mL of water and the FPy-TFP was eluted from PS-2 cartridge with 1.0 mL dichloromethane. An aliquot of 0.5-1 MBq of the eluate was removed for quality control using HPLC: column COSMOSIL 5C18-ARII 6.0ID*150 mm; mobile phase: A: H₂O + 0.1% TFA, B: acetonitrile + 0.1% TFA; method: 0->20 min, 30%->80% B, 20-22 min 80%->95% B, 22-27 min, 95% B, 27-30 min, 95%->30% B, flow rate 2.0 mL/min, retention time 15.2 min. The eluate was concentrated under nitrogen flow at room temperature. To the residue was added a solution of TBS protected alendronate ethyl ester in DMSO (200 µL) and the resulting solution was heated at 50°C for 15 min. Hydrochloric acid aq. (4M, 300 µL) was added to the reaction solution, which was further heated at 100°C for 10 min. The reaction solution was diluted with 1 mL PBS buffer and purified via HPLC: column COSMOSIL 5C18-ARII 6.0ID*150 mm; mobile phase: A: H₂O, B: ethanol; method: 0->20 min, 40%->80% B, 20-22 min 80%->95% B, 22-25 min, 95% B, 25-27 min, 95%->40% B, 27-30 min, 40% B, flow rate 2.0 mL/min, retention time 16.5 ± 0.5 min. The collected fraction was filtered through a 0.2 µM sterile filter and was ready for ex vivo and in vivo application.

### Animal Preparation

Male Wistar rats (weighing 250-400 g) were used for cardiac uptake studies. Anesthesia was induced by 5% isoflurane in pure oxygen and maintained during the experiment by 2% isoflurane.

### PET Imaging and kinetic studies

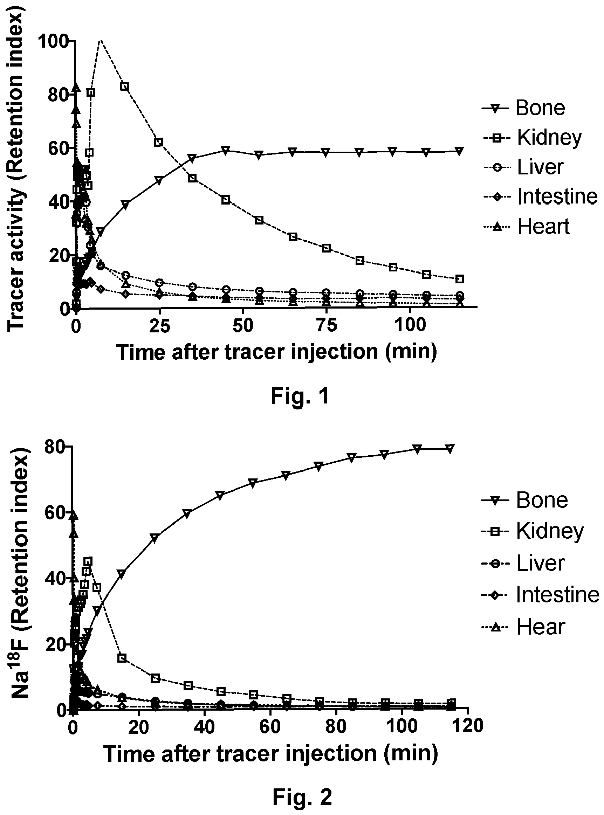

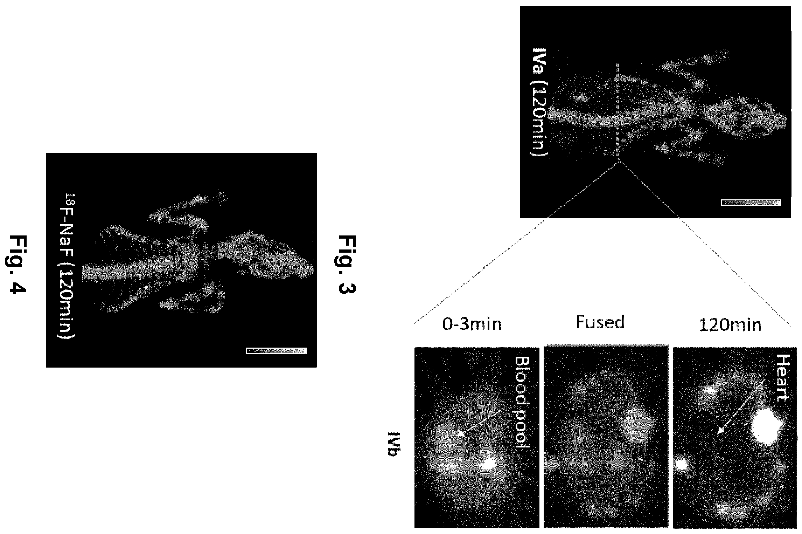

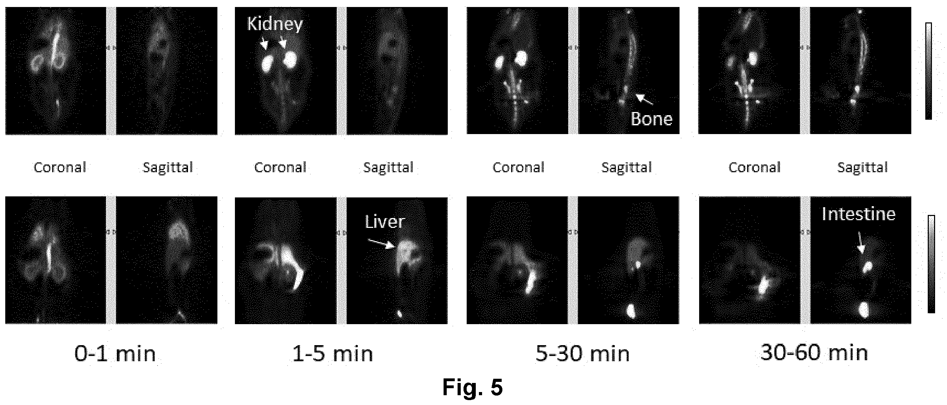

A small-animal PET system (Inveon, Siemens, Erlangen, Germany) was used for the rat studies. A 120-min dynamic scan was started immediately after the injection of **IVa** (10-20 MBq) via the tail vein. The re-constructed images were analyzed using AMIDE imaging software (version 1.0.1). For kinetic studies of rats, the tissue uptake was calculated as percentage of radiotracer retention, from which time-activity curves of different organs, especially bone, kidney, liver, heart and intestine, were generated as illustrated in Figs. 1 to 5.

### Heart tissue binding study

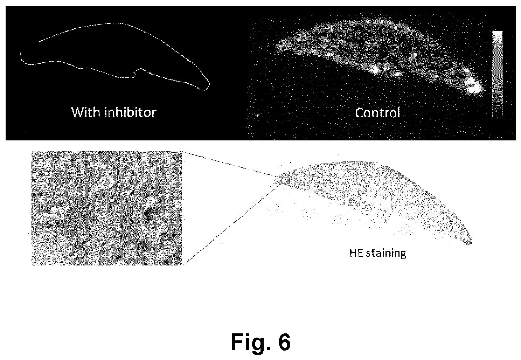

The frozen heart tissue slices were incubated in saline containing **IVa** (approximately 5 MBq) with or without alendronate (final concentration 1 mM) as blocking agent. After incubating for 30 min at 25°C, the slices were rinsed in PBS buffer for 1 min, then four times in water for 2 sec each time. After drying immediately with hairdryer to remove the water and moisture, the slices were exposed to a phosphor imaging plate for 20 min. The images were obtained using a digital autoradiographic system as illustrated in Fig. 6.

## Claims

1. A compound or pharmaceutically acceptable salt thereof for use in a method of diagnosis of cardiac amyloidosis, wherein the compound or pharmaceutically acceptable salt thereof comprises at least one bisphosphonate or bisphosphonate ester group, and
a positron emitting radionuclide Po, wherein Po is attached to the at least one bisphosphonate or bisphosphonate ester group.

2. The compound or a pharmaceutically acceptable salt thereof according to claim 1, wherein the compound or the pharmaceutically acceptable salt thereof is a compound of formula (I) 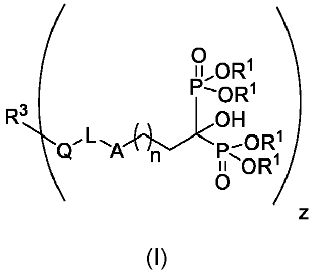 wherein
z is an integer selected from the group consisting of 1, 2, and 3, preferably z is 1; and wherein
each R¹ is independently hydrogen, a pharmaceutically acceptable cation, or a protection group; and wherein
n is an integer selected from the group consisting of 0, 1, 2, and 3; and wherein
A is -NR² , or (4 to 8 membered)heterocyclyl, or (4 to 8 membered)cyclyl, or
squaramide, or (5 to 6 membered)heteroaryl, or phenyl; and wherein
R² is hydrogen or linear or branched (C₁-C₆)alkyl; and wherein
i) L and Q are absent; or wherein
ii) L is a divalent linker group; and wherein
Q is -NH, -NR^{Q}, -N⁺R^{Q}₂X⁻, -O, *N*,*N*-substituted piperazine, or 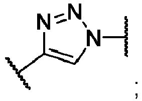 and wherein
X⁻ is a pharmaceutically acceptable anion, preferably X⁻ is F⁻, Cl⁻, Br, I⁻, OH⁻, tosylate, mesylate, triflate, trifluoroacetate, sulfate, acetate, tartrate, benzoate, oxylate, salicylate, or camphorsulfonate; more preferably Br⁻; and wherein
R^{Q} is each independently linear or branched (C₁-C₆)alkyl, preferably each R^{Q} is methyl; and wherein
R³ is a group comprising the positron emitting radionuclide Po.

3. The compound or pharmaceutically acceptable salt thereof according to claim 1 or 2, wherein the positron emitting radionuclide Po is ¹¹C, ¹³N, ¹⁵O, ¹⁸F, ⁶⁴Cu or ⁶⁸Ga,
preferably ¹¹C, ¹⁸F, or ⁶⁸Ga,
more preferably wherein the positron emitting radionuclide Po is ¹⁸F or ⁶⁸Ga.

4. The compound according to claim 2 or 3, wherein each R¹ is
i) a protection group selected from the group consisting of methyl, ethyl, n-propyl, i-propyl, n-butyl, i-butyl, t-butyl, -CH₂-O(CO)-CH₃, -CH₂-O(CO)-CH₂CH₃, -CH₂-O(CO)-CH(CH₃)₂, -CH₂-O(CO)-C(CH₃)₃, and 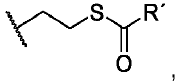 wherein R' is methy, ethyl; preferably wherein
each R¹ is selected from the group consisting of methyl, ethyl, and i-propyl; more preferably wherein each R¹ is methyl or i-propyl; or
ii) wherein each R¹ is
a protection group, wherein two R¹ attached to one phosphonate group together form a structure of 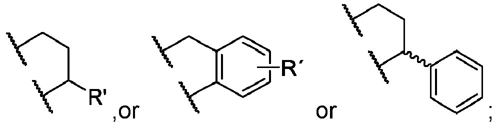 wherein R' is hydrogen or (C₁-C₁₀)alkyl.

5. The compound according to any one of claims 2 to 4, wherein
i) the moiety R³-Q-L-A- is R³-Q-L-NR²-, and wherein
R² is hydrogen, or linear or branched (C₁-C₆)alkyl;
preferably R² is hydrogen, methyl or ethyl;
more preferably R² is hydrogen or methyl; or
ii) the moiety R³-Q-L-A- is 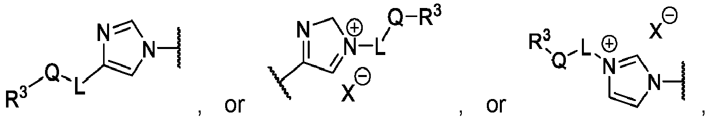 or 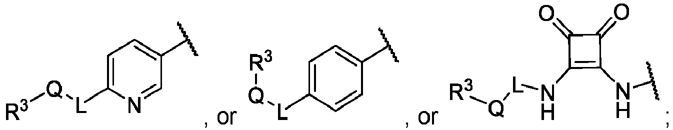
wherein
X⁻ is a pharmaceutically acceptable anion,
preferably X⁻ is F⁻, Cl⁻, Br, I⁻, OH⁻, tosylate, mesylate, triflate, trifluoroacetate, sulfate, acetate, tartrate, benzoate, oxylate, salicylate, or camphorsulfonate; more preferably Br⁻.

6. The compound or pharmaceutically acceptable salt thereof according to any one of claims 2 to 5, wherein
a) L and Q are absent; or wherein
b) L is linear or branched (C₁-C₁₀) alkyl, substituted or unsubstituted phenyl; substituted or unsubstituted (5 to 6 membered)heteroaryl, or (5 to 8 membered)heterocyclyl substituted with (C₁-C₁₀)alkyl, or (C₁-C₆)ω amino acid, or polyethylene glycol, or dipeptide, or tripeptide, or a combination thereof; and wherein
Q is -NH, -NR^{Q}, -N⁺R^{Q}₂X⁻, -O, *N*,*N*-substituted piperazine, or 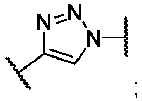 and
wherein
X⁻ is a pharmaceutically acceptable anion, preferably X⁻ is F⁻, Cl⁻, Br, I⁻, OH⁻, tosylate, mesylate, triflate, trifluoroacetate, sulfate, acetate, tartrate, benzoate, oxylate, salicylate, or camphorsulfonate; more preferably Br⁻; and wherein
R^{Q} is each independently linear or branched (C₁-C₆)alkyl, preferably each R^{Q} is methyl.

7. The compound or pharmaceutically acceptable salt thereof according to any one of claims 2 to 6, wherein
a) the moiety R³-Q-L- is 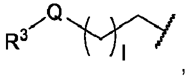 wherein
I is an integer selected from the group consisting of 1, 2, 3, 4, 5, 6, and 7, preferably wherein I is 1, 2, or 3; and wherein
Q is -NH, -NR^{Q}, -N⁺R^{Q}₂ X⁻, -O, *N*,*N*-substituted piperazine, or 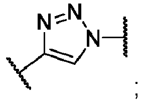 wherein
X⁻ is a pharmaceutically acceptable anion, preferably X⁻ is F⁻, Cl⁻, Br⁻, I⁻, OH⁻, tosylate, mesylate, triflate, trifluoroacetate, sulfate, acetate, tartrate, benzoate, oxylate, salicylate, or camphorsulfonate; more preferably Br⁻; and wherein
R^{Q} is each independently linear or branched (C₁-C₆)alkyl, preferably each R^{Q} is methyl; or
b) the moiety R³-Q-L- is 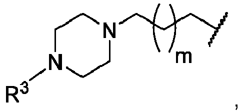 wherein
m is 1, 2, or 3; or
c) the moiety R³-Q-L- is 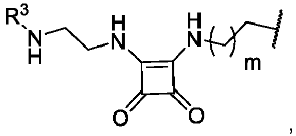 wherein
m is 1, 2, or 3.

8. The compound or pharmaceutically acceptable salt thereof according to any one of claims 2 to 7, wherein R³ is
i) 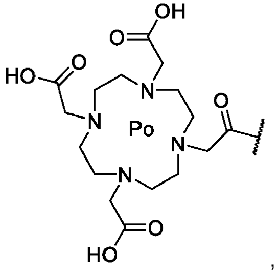 wherein Po is ⁶⁴Cu or ⁶⁸Ga, preferably ⁶⁸Ga; or
ii) 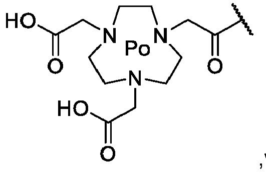 ,wherein Po is ⁶⁴Cu or ⁶⁸Ga, preferably ⁶⁸Ga; or
iii) 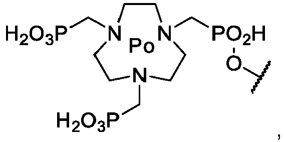 wherein Po is ⁶⁴Cu or ⁶⁸Ga, preferably ⁶⁸Ga; or
iv) 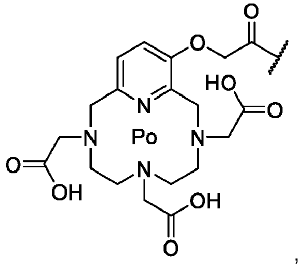 wherein Po is ⁶⁴Cu or ⁶⁸Ga, preferably ⁶⁸Ga; or
v) 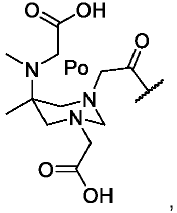 wherein Po is ⁶⁴Cu or ⁶⁸Ga, preferably ⁶⁸Ga; or
vi) 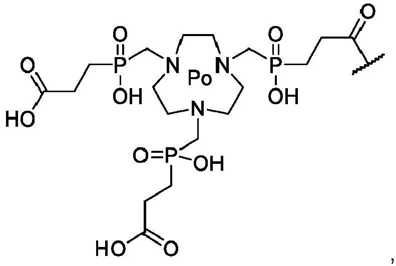 wherein Po is ⁶⁴Cu or ⁶⁸Ga, preferably ⁶⁸Ga; or
vii) 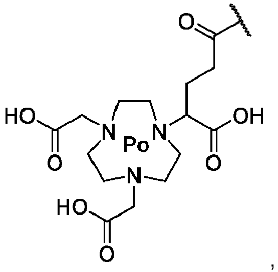 wherein Po is ⁶⁴Cu or ⁶⁸Ga, preferably ⁶⁸Ga; or
viii) 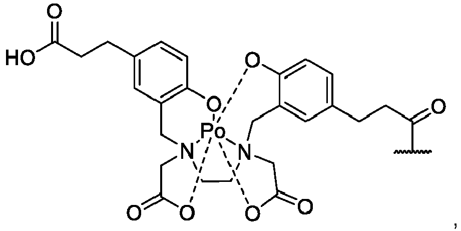 wherein Po is ⁶⁴Cu or ⁶⁸Ga, preferably ⁶⁸Ga; or
ix) 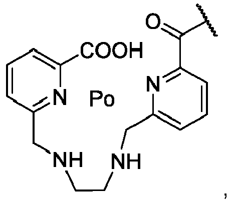 wherein Po is ⁶⁴Cu or ⁶⁸Ga, preferably ⁶⁸Ga; or
x) 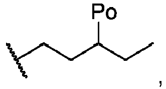 wherein Po is ¹⁸F or -O¹¹CH₃, preferably ¹⁸F; or
xi) 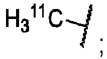 or
xii) 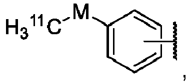 wherein M is O, or -NH, or CH₂O; or
xiii) 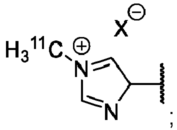 or
xiv) 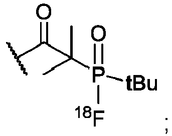 or
xv) 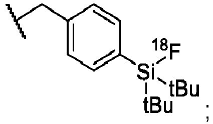 or
xvi) 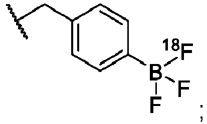 or
xvii) 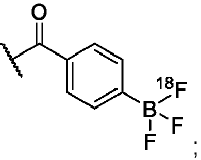 or
xviii) 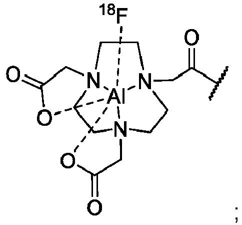 or
xix) 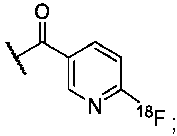 or
xx)

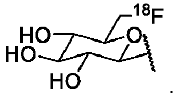

9. The compound or pharmaceutically acceptable salt thereof according to any one of claims 1 to 8, wherein the compound or pharmaceutically acceptable salt thereof is a compound of formula (II) 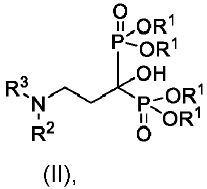
R³ is
i) 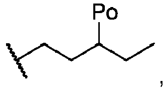 wherein Po is ¹⁸F or -O¹¹CH₃, preferably ¹⁸F; or
ii) 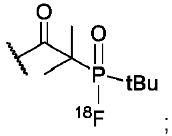 or
iii) 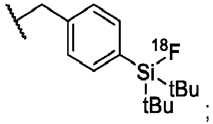 or
iv) 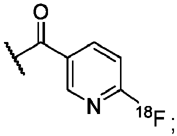 preferably wherein
R³ is 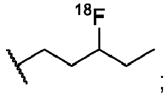 and wherein
R² is hydrogen or linear or branched (C₁-C₆)alkyl,
preferably R² is hydrogen or methyl,
more preferably R² is methyl; and wherein
each R¹ is independently selected from the group consisting of hydrogen, linear or branched (C₁-C₆)alkyl, and -CH₂-O-CO-O-(C₁-C₆)alkyl,
preferably wherein each R¹ is hydrogen, methyl, ethyl, i-propyl, or -O(CO)-C(CH₃)₃,
more preferably each R¹ is hydrogen, methyl, or -CH₂-O(CO)-C(CH₃)₃, yet more preferably each R¹ is hydrogen or -CH₂-O(CO)-C(CH₃)₃; most preferably wherein
the compound or pharmaceutically acceptable salt thereof has the structure (IIa) or (IIb) 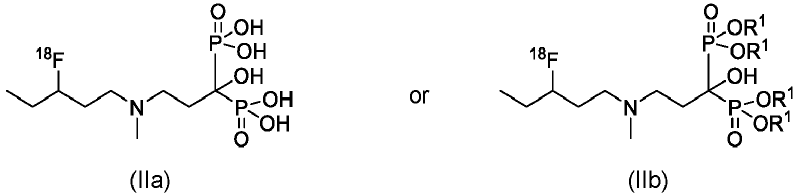 wherein R¹ is methyl or ethyl.

10. The compound or pharmaceutically acceptable salt thereof according to any one of claims 1 to 8, wherein the compound or the pharmaceutically acceptable salt thereof is
a) a compound of formula (III) 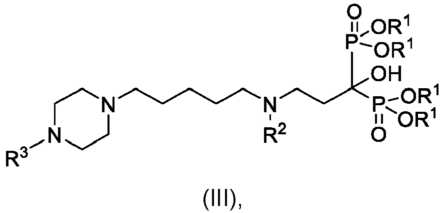 wherein R³ is
i) 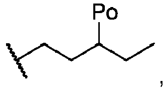 wherein Po is ¹⁸F or -O¹¹CH₃, preferably ¹⁸F; or
ii) 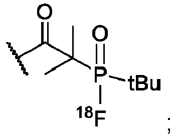 or
iii) 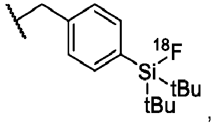 or
iv) 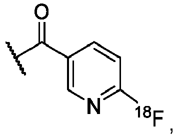 preferably wherein
R³ is 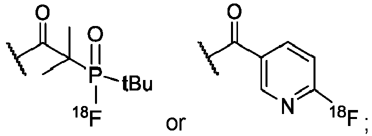 and wherein
R² is hydrogen or linear or branched (C₁-C₆)alkyl,
preferably R² is hydrogen or methyl,
more preferably R² is methyl; and wherein
each R¹ is independently selected from the group consisting of hydrogen, linear or branched (C₁-C₆)alkyl, and -CH₂-O-CO-O-(C₁-C₆)alkyl,
preferably each R¹ is hydrogen, methyl, ethyl, or i-propyl,
more preferably each R¹ is hydrogen, methyl, or i-propyl,
yet more preferably each R¹ is hydrogen or methyl; most preferably wherein the compound or pharmaceutically acceptable salt thereof has the structure (IIIa) or (IIIb) 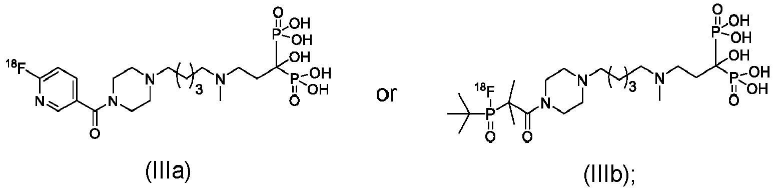 or
b) a compound of formula (V) 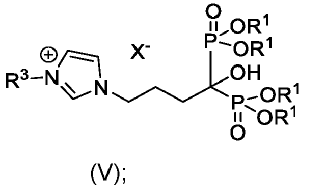 wherein
X⁻ is a pharmaceutically acceptable anion,
preferably X⁻ is F⁻, Cl⁻, Br, I⁻, or OH⁻;
more preferably Br; and wherein
R³ is
i) 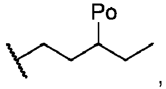 wherein Po is ¹⁸F or O¹¹CH₃, preferably ¹⁸F; or
ii) 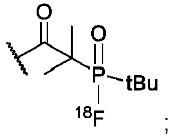 or
iii) 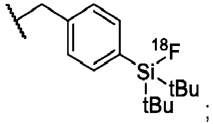 or
iv) 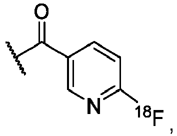 preferably wherein
R³ is 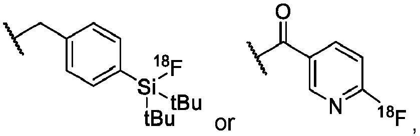 more preferably
R³ is 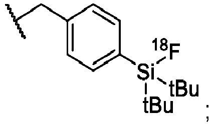 and wherein
each R¹ is independently selected from the group consisting of hydrogen, linear or branched (C₁-C₆)alkyl, and -CH₂-O-CO-O-(C₁-C₆)alkyl,
preferably wherein each R¹ is hydrogen, methyl, ethyl, or i-propyl,
more preferably each R¹ is hydrogen, methyl, or i-propyl,
yet more preferably each R¹ is hydrogen or methyl; most preferably
wherein the compound or pharmaceutically acceptable salt thereof has the structure (Va) 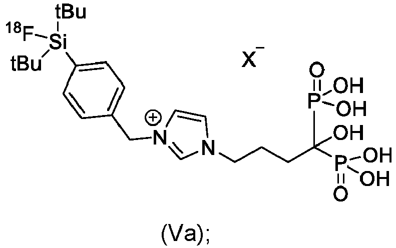 or
c) a compound of formula (VI) 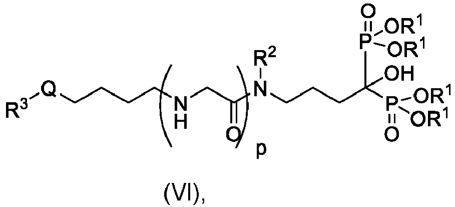 wherein
Q is -NH, -NR^{Q}, -N⁺R^{Q}₂X⁻, or -O; wherein
X⁻ is a pharmaceutically acceptable anion, preferably X⁻ is F⁻, Cl⁻, Br, I⁻, OH⁻, tosylate, mesylate, triflate, trifluoroacetate, sulfate, acetate, tartrate, benzoate, oxylate, salicylate, or camphorsulfonate; more preferably Br; and wherein R^{Q} is linear or branched (C₁-C₆)alkyl; and wherein
p is 0 or 1; and wherein
R³ is
i) 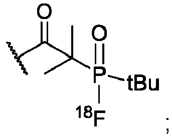 or
ii) 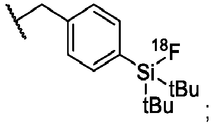 or
iii) 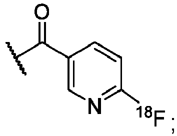 and wherein
R² is hydrogen or linear or branched (C₁-C₆)alkyl,
preferably R² is hydrogen or methyl; and wherein
each R¹ is independently selected from the group consisting of hydrogen, linear or branched (C₁-C₆)alkyl, and -CH₂-O-CO-O-(C₁-C₆)alkyl,
preferably wherein each R¹ is hydrogen, methyl, ethyl, i-propyl, or -O(CO)-C(CH₃)₃,
more preferably each R¹ is hydrogen, or methyl, yet more preferably each R¹ is hydrogen; most preferably
wherein the compound or pharmaceutically acceptable salt thereof has the structure (VIa), (VIb), or (VIc) 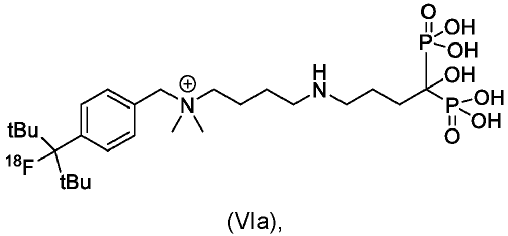 or 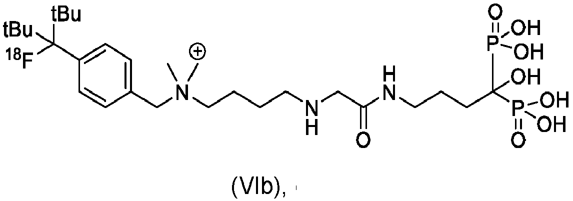 or 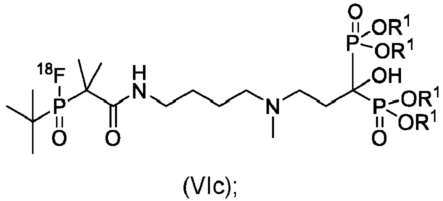 or
d) a compound of formula (VII) 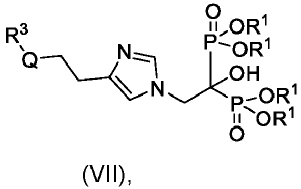 wherein
Q is -NH, -NR^{Q}, -N⁺R^{Q}₂X⁻, *N,N*-substituted piperazine or -O; and wherein
X⁻ is a pharmaceutically acceptable anion, preferably X⁻ is F⁻, Cl⁻, Br⁻, I⁻, or OH⁻; more preferably Br⁻; and wherein
R^{Q} is each independently linear or branched (C₁-C₆)alkyl, preferably each R^{Q} is methyl; preferably Q is -NH; and wherein
R³ is
i) 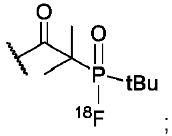 or
ii) 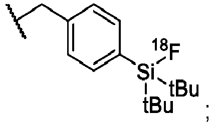 or
iii) 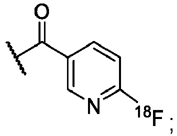 and wherein
each R¹ is independently selected from the group consisting of hydrogen, linear or branched (C₁-C₆)alkyl, and -CH₂-O-CO-O-(C₁-C₆)alkyl,
preferably wherein each R¹ is hydrogen, methyl, ethyl, i-propyl, or -O(CO)-C(CH₃)₃,
more preferably each R¹ is hydrogen, or methyl, yet more preferably each R¹ is hydrogen; most preferably
wherein the compound or pharmaceutically acceptable salt thereof has the structure (VIIa)

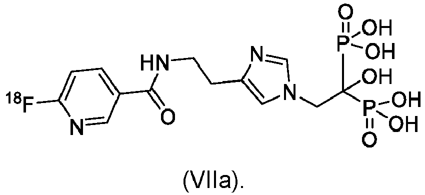

11. The compound or pharmaceutically acceptable salt thereof according to any one of claims 1 to 8, wherein the compound or the pharmaceutically acceptable salt thereof is a compound of formula (IV) 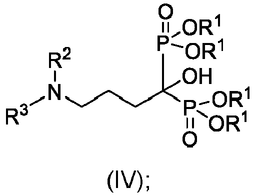 wherein R³ is
i) 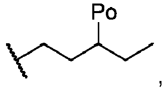 wherein Po is ¹⁸F or -O¹¹CH₃, preferably ¹⁸F; or
ii) 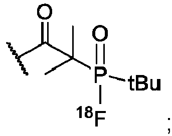 or
iii) 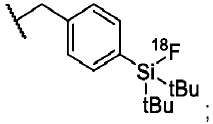 or
iv) 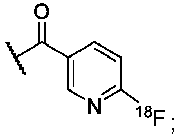 preferably wherein
R³ is 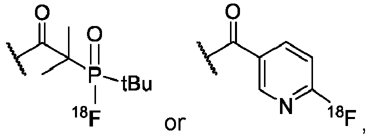 more preferably wherein
R³ is 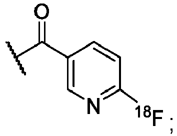 and wherein
R² is hydrogen or linear or branched (C₁-C₆)alkyl,
preferably R² is hydrogen or methyl,
more preferably R² is hydrogen; and wherein
each R¹ is independently selected from the group consisting of hydrogen, linear or branched (C₁-C₆)alkyl, and -CH₂-O-CO-O-(C₁-C₆)alkyl,
preferably wherein each R¹ is hydrogen, methyl, ethyl, or i-propyl,
more preferably each R¹ is hydrogen, methyl, or i-propyl,
yet more preferably each R¹ is hydrogen or methyl; most preferably wherein the compound or pharmaceutically acceptable salt thereof has the structure (IVa) or (IVb) 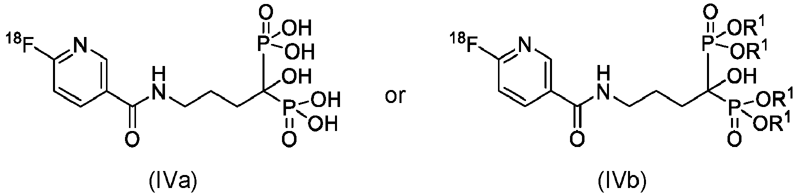
wherein R¹ is methyl or ethyl, preferably ethyl.

12. The compound or pharmaceutically acceptable salt thereof according to any one of the preceding claims, wherein the compound is not 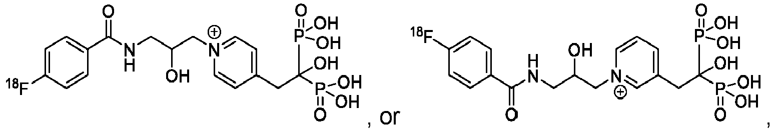 or 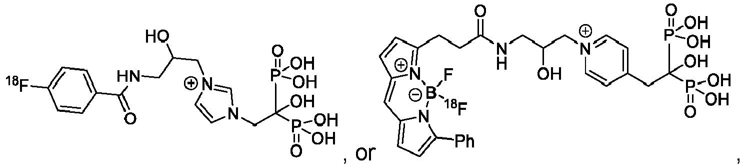 or 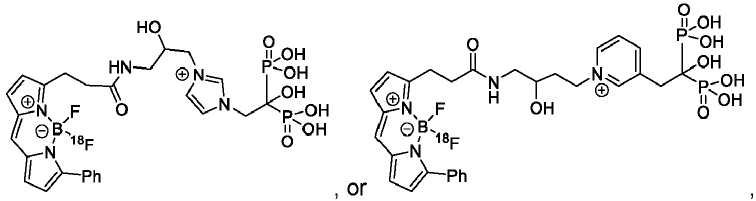 or 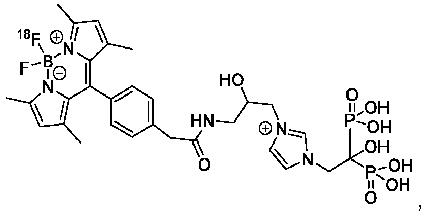 or 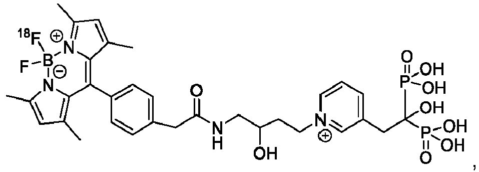 or

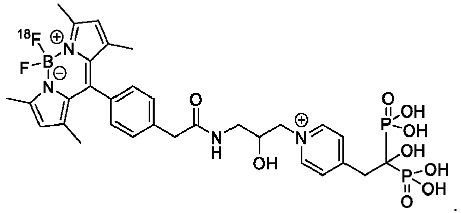

13. A method of preparation of a compound according to any one of claims 1 to 12, the method comprising the steps of
a) providing a compound of formula (Ia) 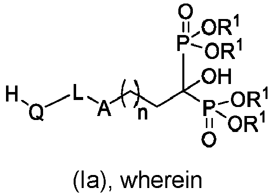
each R¹ is independently hydrogen, a pharmaceutically acceptable cation, or a protection group; and wherein
n is an integer selected from the group consisting of 0, 1, 2, and 3; and
wherein
A is -NR², or (4 to 8 membered)heterocyclyl, or (4 to 8 membered)cyclyl, or squaramide, or (5 to 6 membered)heteroaryl, or phenyl; and wherein
R² is hydrogen or linear or branched (C₁-C₆)alkyl; and wherein
L and Q are absent; or wherein
L is a divalent linker group, and wherein
Q is -NH, -NR^{Q}, -N⁺R^{Q}₂ X⁻, -O, or *N,N*-substituted piperazine; wherein R^{Q} is each independently linear or branched (C₁-C₆)alkyl, preferably each R^{Q} is methyl; and
b) providing a compound of formula R³-Y, wherein R³-Y is
i) 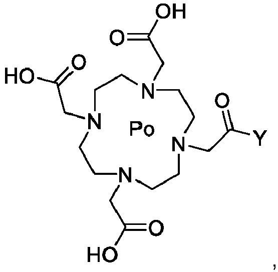 wherein Po is ⁶⁴Cu or ⁶⁸Ga, preferably ⁶⁸Ga; or
ii) 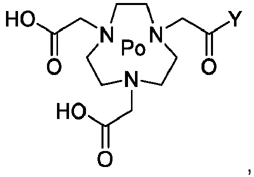 wherein Po is ⁶⁴Cu or ⁶⁸Ga, preferably ⁶⁸Ga; or
iii) 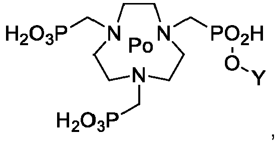 wherein Po is ⁶⁴Cu or ⁶⁸Ga, preferably ⁶⁸Ga; or
iv) 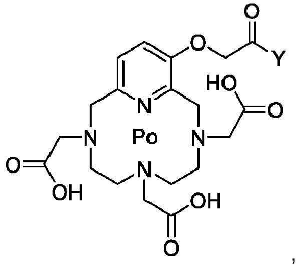 wherein Po is ⁶⁴Cu or ⁶⁸Ga, preferably ⁶⁸Ga; or
v) 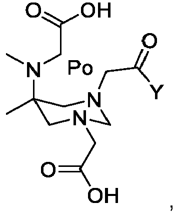 wherein Po is ⁶⁴Cu or ⁶⁸Ga, preferably ⁶⁸Ga; or
vi) 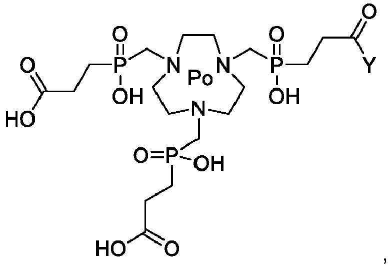 wherein Po is ⁶⁴Cu or ⁶⁸Ga, preferably ⁶⁸Ga; or
vii) 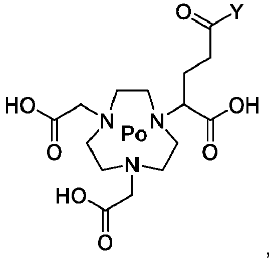 wherein Po is ⁶⁴Cu or ⁶⁸Ga, preferably ⁶⁸Ga; or
viii) 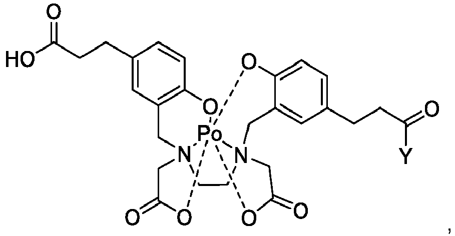 wherein Po is ⁶⁴Cu or ⁶⁸Ga, preferably ⁶⁸Ga; or
ix) 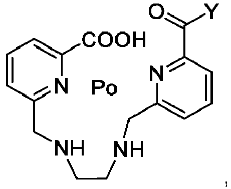 wherein Po is ⁶⁴Cu or ⁶⁸Ga, preferably ⁶⁸Ga; or
x) 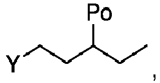 wherein Po is ¹⁸F or -O¹¹CH₃, preferably ¹⁸F; or
xi) Y-¹¹CH₃, or HO-¹¹CH₃;
xii) 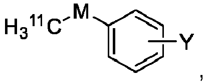 wherein M is O, or -NH, or CH₂O; or
xiii) 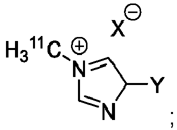 or
xiv) 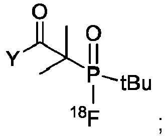 or
xv) 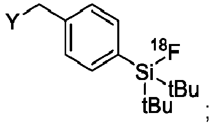 or
xvi) 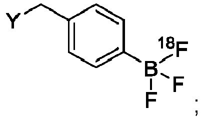 or
xvii) 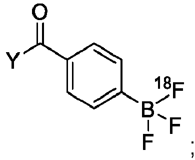 or
xviii) 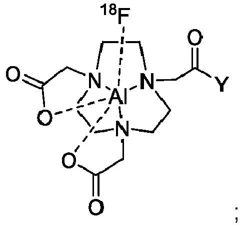 or
xix) 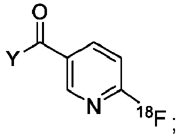 and wherein
Y is an electron withdrawing group,
preferably Y is selected from the group consisting of -Cl, -Br, -O-C₆F₅H, -O(CO)-(C₁-C₆)alkyl, and -OH; and
c) reacting the compounds of formula (Ia) and any one of R³-Y at a temperature of from about 60 °C to about 140 °C, wherein
when Y is -OH, step b) further comprises providing dry DMF and a coupling agent selected from the group consisting of EDC, HATU, HOBt, HBTU, and HATU; most preferably
wherein step c) is performed for a period of from about 1 min to about 60 min; and/or
wherein step c) is performed in dry DMF or dry DMSO.

14. A method of preparation of a compound or pharmaceutically acceptable salt thereof according to any one of claims 1 to 12, the method comprising the steps of
a) providing a compound of formula (Ib) 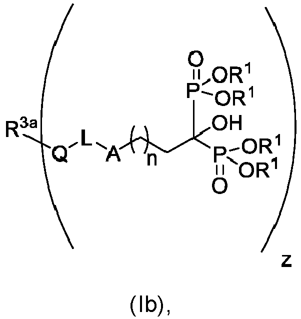 wherein
z is 1, 2, or 3, preferably z is 1; and wherein
each R¹ is independently hydrogen, a pharmaceutically acceptable cation, or a protection group; and wherein
n is an integer selected from the group consisting of 0, 1, 2, and 3; and wherein
A is -NR², or (4 to 8 membered)heterocyclyl, or (4 to 8 membered)cyclyl, or
squaramide, or (5 to 6 membered)heteroaryl, or phenyl; and wherein
R² is hydrogen or linear or branched (C₁-C₆)alkyl; and wherein
L and Q are absent; or
L is a divalent linker group; and wherein
Q is -NH, -NR^{Q}, -N⁺R^{Q}₂ X⁻, -O, or *N,N*-substituted piperazine; wherein R^{Q} is each independently linear or branched (C₁-C₆)alkyl, preferably each R^{Q} is methyl; and wherein
R^{3a} is
i) 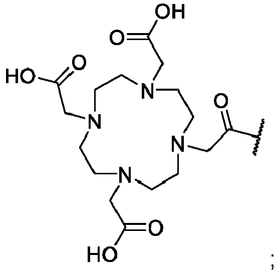 or
ii) 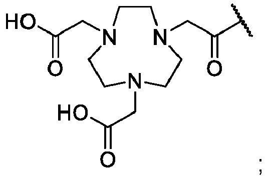 or
iii) 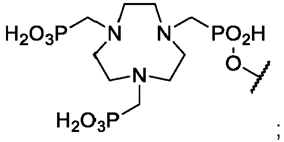 or
iv) 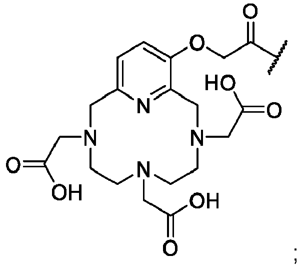 or
v) 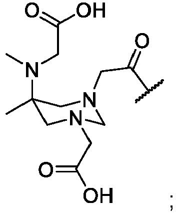 or
vi) 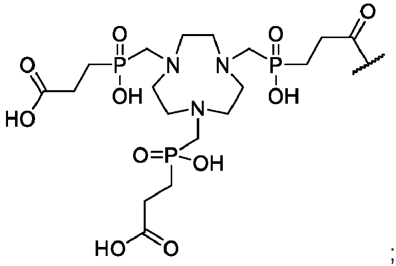 or
vii) 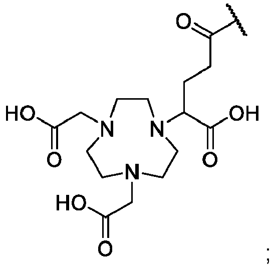 or
viii) 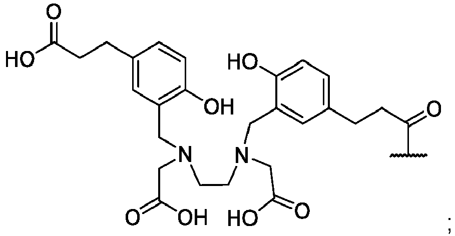 or
ix) 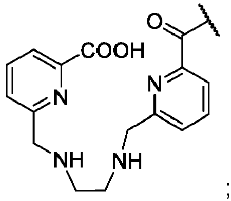 or
x) 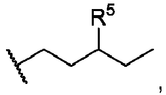 wherein R⁵ is OTs, OMs, OTf, or I; or
xi) 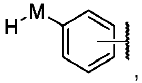 wherein M is O, or -NH, or CH₂O; or
xii) 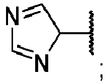 or
xiii) 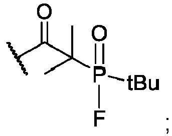 or
xiv) 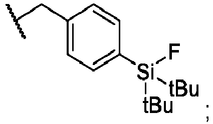 or
xv) 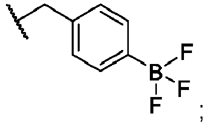 or
xvi) 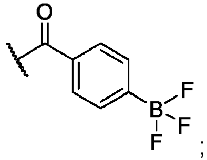 or
xvii) 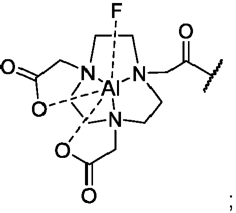 or
xviii) 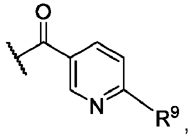 wherein R⁹ is an ammonium salt, or H; and
b) reacting the compound of formula (Ib) with a group Po* comprising th positron emitting radionuclide Po, wherein
Po* is -¹¹CH₃, ¹³N, ¹⁵O ¹⁸F⁻, ⁶⁴Cu or ⁶⁸Ga,
preferably -¹¹CH₃, ¹⁸F⁻, and ⁶⁸Ga,
more preferably ¹⁸F⁻ or ⁶⁸Ga; most preferably
wherein step b) is performed for a period of time of from about 1 min to about 60 min; and/or
wherein step b) is performed at a temperature of from about 80 °C to about 140 °C; and/or
wherein step b) is performed in dry DMF or dry DMSO.

15. A method of preparation of a compound or pharmaceutically acceptable salt thereof according to any one of claims 1 to 12, the method comprising the steps of
a) providing a compound of formula (Ic) 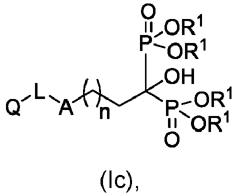 wherein
each R¹ is independently hydrogen, a pharmaceutically acceptable cation, or a protection group; and wherein
n is an integer selected from the group consisting of 0, 1, 2, and 3; and wherein
A is -NR², or (4 to 8 membered)heterocyclyl, or (4 to 8 membered)cyclyl, or
squaramide, or (5 to 6 membered)heteroaryl, or phenyl; and wherein
R² is hydrogen or linear or branched (C₁-C₆)alkyl; and wherein
L is (C₁-C₁₀) alkyl, and Q is -C≡CH; and
b) providing a compound of formula 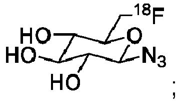 and
c) reacting the compounds of formula (Ic) and step b) at a temperature of from about 60 °C to about 140 °C in the presence of a Cu(I) salt; preferably
wherein step c) is performed for a period of time of from about 1 min to about 60 min; and/or
wherein step c) is performed at a temperature of from about 80 °C to about 140 °C; and/or
wherein step c) is performed in dry DMF or dry DMSO.
